# EUROPÄISCHE PATENTSCHRIFT

(11) **EP 0 534 102 B1**
(45) Veröffentlichungstag und Bekanntmachung des Hinweises auf die Patenterteilung: **19.12.2001**
(21) Anmeldenummer: 92113397.1
(22) Anmeldetag: 06.08.1992
(51) Int. Cl.: C07K 14/45, C07K 16/00, C12N 15/38, G01N 33/543, G01N 33/569, A61K 39/245

(54) **HCMV-spezifische Peptide und ihre Verwendung**
Human cytomegalovirus specific peptides and use thereof
Peptides spécifiques pour le cytomégalovirus humain et leurs utilisations

(30) Priorität: 29.08.1991 DE 4128684
(43) Veröffentlichungstag der Anmeldung: 31.03.1993
(73) Patentinhaber: Dade Behring Marburg GmbH, 35001 Marburg (DE)
(72) Erfinder: Stüber, Werner, W- 3551 Lahntal 3 (DE); Wieczorek, Leszek, W-3550 Marburg 21 (DE); Ziegelmaier, Robert, W-3550 Marburg (DE)

(56) Entgegenhaltungen:
- EP-A- 0 252 531
- EP-A- 0 268 014
- EP-A- 0 271 201
- WO-A-89/01628

## Beschreibung

Die Erfindung betrifft Peptide zur immunchemischen Bestimmung von humanen Cytomegalovirus (HCMV)-spezifischen Antikörpern und HCMV-Antigenen sowie für diese Verfahren geeignete Mittel und ihre Verwendung.

Bisher wurde in der Diagnostik zur Überprüfung der HCMV-Immunitätslage beispielsweise ein Enzymimmunotest benutzt. In diesem Test wurden Antikörper, die spezifisch gegen das HCMV gerichtet sind, nachgewiesen. Als Antigen wurde Virusmaterial eingesetzt, das im allgemeinen in einer aufwendigen Zellkultur auf humanen Fibroblasten vermehrt und nach Aufbereitung auf eine diagnostisch verwendbare Oberfläche, z. B. eine ELISA-Mikrotiterplatte, ausgebracht wurde.

Die Gewinnung von HCMV-Antigen für einen Antikörper-nachweis aus der Zellkultur ist im allgemeinen sehr zeit- und kostenaufwendig und für die damit beschäftigten Personen mit einer möglichen Infektionsgefahr verbunden. Für die Verwendung dieses Antigens in einem immunologisch-diagnostischen Test muß das Virus aus dem Medium der Zellkultur oder sogar aus den Zellen selbst gewonnen werden oder zumindest in einer Form präsentiert werden, die eine immunologische Reaktion ermöglicht. Da eine weitere Reinigung dieses Materials durch biochemische Verfahren sehr aufwendig und verlustreich ist, wird beispielsweise zellgebundenes Virusmaterial nur durch Ultrabeschallung freigesetzt und direkt nach Verdünnung für die Beschichtung von z. B. Mikrotitrationsplatten eingesetzt. Dabei werden nicht nur virusspezifische Strukturen an die Oberflächen der Mikrotiterplattenkavitäten gebunden, sondern zum größeren Teil auch zellspezifische Proteine. Diese können wiederum bei bestimmten Krankheiten, beispielsweise Autoimmunerkrankungen, leicht zu falsch-positiven Resultaten und folglich zu einer Fehldiagnose führen. Zur Bestimmung der Anzahl der falsch-positiven Signale wird daher als Kontrolle im allgemeinen zusätzlich Untersuchungsmaterial aus nichtinfizierten Zellkulturen verwendet. Dadurch verdoppelt sich zwangsweise der Testaufwand und die Kosten je Probe.

Eine deutliche Verbesserung des beschriebenen Verfahrens stellt die Verwendung von rekombinanten Proteinen dar, die in einem heterologen System, z. B. in Escherichia coli, in großer Ausbeute hergestellt werden können.
Durch die Klonierung und Expression definierter Teilbereiche des Virus ist eine mögliche HCMV-Infektion im allgemeinen ausgeschlossen. Zusätzlich ist die Möglichkeit einer auf spezifische Virusproteine ausgerichteten Differentialdiagnostik gegeben. Für einen Einsatz von rekombinanten Proteinen in immunologischen Tests muß jedoch gewährleistet werden, daß keine kontaminierenden Bestandteile der Wirtszelle zu falsch-positiven Reaktionen mit Serumproben führen. Hier sind spezielle, meist sehr aufwendige Reinigungsverfahren notwendig, um diese Qualität zu erreichen.

Weiterhin müssen die rekombinanten Proteine immunologisch reaktive Epitope tragen, die den Immunstatus eindeutig bestimmen und eine diagnostische Relevanz bzw. eine Relevanz für die Herstellung eines Schutzstatus (Vakzine) besitzen. In den meisten Fällen sind diese Epitope nicht in nativer Form mit rekombinanten Techniken herzustellen, weil sie für den Großteil der verwendeten Expressionssysteme Fremdproteine darstellen, die schnell proteolytisch abgebaut werden. Aus diesem Grund werden sie im Regelfall als Hybridproteine exprimiert, wobei ein wirtseigenes Protein, z. B. die β-Galaktosidase bei Escherichia coli als Fusionspartner das Expressionsprodukt stabilisiert. Allerdings kann dieser Fusionsfremdanteil, bedingt durch die folgenden Aufreinigungsschritte, Anlaß zu falsch positiven Reaktionen geben, die die diagnostische Verwendung von rekombinanten Proteinen erschweren.

Für HCMV wurde nun ein Protein identifiziert, das im allgemeinen für die Bestimmung des Immunstatus geeignet ist. Von diesem Protein, dem Protein pp150, ist sowohl die gesamte DNA-Sequenz, wie auch ein 162 aminosäurenlanger Abschnitt (das XhoI-PstI Restriktionsfragment, als Plasmid kloniert pXP1 genannt), bekannt, der die für eine eindeutige Diagnostik relevanten Sequenzen enthält (Jahn, G. et al. (1987), J. Virol. 61, 1358-1367).

J. Novak et al. (J. Novak et al. (1991), J. Gen. Virol. 72, 1409-1413) fanden nun auf dem gesamten pp150 Protein mit Hilfe von synthetisch hergestellten Peptiden nur drei immunreaktive Bereiche, von denen zwei Bereiche zwischen den Schnittstellen der Restriktionsendonukleasen XhoI und PstI (XP1 Region, Tab. 1) liegen.

Die Aminosäuren werden in der Tab. 1 als Ein-Buchstaben-Code nach folgendem Schlüssel wiedergegeben: Ala = A, Arg = R, Asn = N, Asp = D, Cys = C, Gln = Q, Glu = E, Gly = G, His = H, Ile = I, Leu = L, Lys = K, Met = M, Phe = F, Pro = P, Ser = S, Thr = T, Trp = W, Tyr = Y und Val = V.

Der vorliegenden Erfindung lag nun die Aufgabe zugrunde, einen Test zu entwickeln, mit dem man HCMV-Infektionen möglichst frühzeitig und mit hoher Spezifität nachweisen kann.

Es wurde nun überraschenderweise gefunden, daß die von Novak et al. gefundenen immunreaktiven Bereiche auf der XP1 Region nicht bestätigt werden konnten, statt dessen jedoch wurden drei von Novak et al. unterschiedliche immunreaktive Bereiche gefunden. Zusätzlich fand man, daß eine praktisch vollständige Erfassung des Immunstatus insbesondere durch die Verwendung aller drei erfindungsgemäßen Epitope in einem Immunotest möglich ist.

Ein Gegenstand der Erfindung sind daher Peptide, die spezifisch mit Antikörpern gegen HCMV reagieren, dadurch gekennzeichnet, daß sie mindestens eine der folgenden Aminosäuresequenzen enthalten: ASe-gly ala gly ala ala ile leu-BSm (Peptid 1) CSₙ-arg ala trp ala leu -DSₒ          (Peptid 2) und/oder ES_p-ala ser arg asp ala ala-FS_q (Peptid 3), wobei AS, BS, CS, DS, ES und FS unabhängig voneinander eine beliebige Aminosäure bedeuten und
e unabhängig voneinander ganze Zahlen von 0 bis 22,
m " " " " " 0 bis 25,
n und 0 " " " " " 0 bis 18
   und
p und q " " " " " 0 bis 11
   sind.

### Bevorzugte Peptide sind

### Zu Peptid 1:

1.1
ala tyr lys phe glu gln pro thr leu thr phe gly ala gly
val asn val pro ala gly ala gly ala ala ile leu
1.2
phe glu gln pro thr leu thr phe gly ala gly val asn val
pro ala gly ala gly ala ala ile leu thr pro thr pro val
1.3
phe glu gln pro thr leu thr phe gly ala gly val asn val
pro ala gly ala gly ala ala ile leu thr pro thr pro val
asn pro ser thr ala
1.4
phe glu gln pro thr thr leu thr phe gly ala gly val asn
val pro ala gly ala gly ala ala ile leu thr pro thr pro
val asn pro ser thr ala pro ala pro ala
1.5
pro thr leu thr phe gly ala gly val asn val pro ala gly
ala gly ala ala ile leu thr pro thr pro val asn pro ser
thr ala pro ala pro ala
1.6
pro thr leu thr phe gly ala gly val asn val pro ala gly
ala gly ala ala ile leu thr pro thr pro val asn pro ser
thr ala pro ala pro ala pro NH2
1.7
val asn val pro ala gly ala gly ala ala ile leu thr pro
thr pro val asn pro
1.8
pro ala gly ala gly ala ala ile leu thr pro thr pro val
asn pro ser thr ala pro ala
1.9
val asn val pro ala gly ala gly ala ala ile leu thr pro
thr pro val asn pro ser thr ala pro ala pro ala pro thr
pro thr phe

1.10
pro ala gly ala gly ala ala ile leu thr pro thr pro val
asn pro ser thr ala pro ala pro ala pro thr pro thr phe
ala gly thr
1.11
gly ala gly val asn val pro ala gly ala gly ala ala ile
leu thr pro thr pro val asn pro ser thr ala pro ala pro
ala pro NH2
1.12
gly ala gly ala ala ile leu thr pro thr pro val asn pro
ser thr ala pro ala pro ala pro thr pro thr phe ala gly
thr gln thr pro
1.13
ala tyr lys phe gly gln pro thr leu thr phe gly ala gly
val asn val pro ala gly ala gly ala ala ile leu thr pro
thr pro val asn pro ser thr ala pro ala pro ala pro
1.14
ala tyr lys phe glu gln pro thr leu thr phe gly ala gly
val asn val pro ala gly ala gly ala ala ile leu thr pro
thr pro val asn pro ser thr ala pro ala pro ala pro thr
pro thr phe ala gly thr gln thr pro
1.15
ala tyr lys phe glu gln pro thr leu thr phe gly ala gly
val asn val pro ala gly ala gly ala ala ile leu thr pro
thr pro val asn pro ser thr ala pro ala pro ala pro NH2
1.16
gly ala gly val asn val pro ala gly ala gly ala ala ile
leu thr pro thr pro val asn pro ser thr ala pro ala pro
ala

### Zu Peptid 2:

2.1
asp met asn pro ala asn trp pro arg glu arg ala trp ala
leu lys asn pro his leu ala tyr asn pro phe
2.2
asp met asn pro ala asn trp pro arg glu arg ala trp ala
leu lys asn pro his leu
2.3
pro ala asn trp pro arg glu arg ala trp ala leu lys asn
pro his leu
2.4
pro ala asn trp pro arg glu arg ala trp ala leu lys asn
pro his leu ala tyr asn
2.5
trp pro arg glu arg ala trp ala leu lys asn pro his leu
ala tyr asn pro phe arg
2.6
glu arg ala trp ala leu lys asn pro his leu ala tyr asn
pro phe arg met pro thr thr
2.7
asp met asn pro ala asn trp pro arg glu arg ala trp ala
leu
2.8
ala asn trp pro arg glu arg ala trp ala leu lys asn pro
his leu ala
2.9
asp met asn pro ala asn trp pro arg glu arg ala trp ala
leu lys asn pro his leu ala tyr asn pro phe arg met pro
thr thr ser thr ala
2.10
arg ala trp ala leu lys asn pro his leu ala tyr asn pro
phe

### Zu Peptid 3:

3.1
pro arg ala ala val thr gln thr ala ser arg asp ala ala
3.2
ala ser arg asp ala ala asp glu val trp ala leu arg asp
gln thr ala

Davon sind insbesondere folgende Peptide bevorzugt:

### Zu Peptid 1:

1.3, 1.4, 1.6, 1.11, 1.13

### Zu Peptid 2:

2.1, 2.5, 2.9 und/oder 2.10

### Zu Peptid 3:

### 3.2

Die Ausdrücke Peptide und Polypeptide werden im Sinne der Erfindung als gleichwertig für Peptide und Proteine mit bis zu etwa 80 AS verwendet.

Unter immunreaktiven Peptiden versteht man im allgemeinen Peptide mit mindestens einem Epitop, wobei die Mindestlänge der Peptide im allgemeinen ungefähr 6, vorzugsweise ungefähr 8 - 10 Aminosäuren ist.

Häufig ist es vorteilhaft, Peptide vielfältig zu derivatisieren, wie z. B. durch Amino-terminale bzw. Carboxy-terminale Angliederung einer oder mehrerer Aminosäuren, vorzugsweise Cystein, um beispielsweise das Verknüpfen von Peptiden untereinander oder an einen Träger zu erreichen. Die Verlängerungen betragen im allgemeinen 1 bis 40, vorzugsweise 1 bis 20, insbesondere 1 bis 10 Aminosäuren. Weitere Beispiele sind Thioglykolsäure-Amidierung, Carboxy-terminale Amidierung, wie z. B. mit ammonia oder Methylamin. Derartige Modifikationen können die Nettoladung des Polypeptids verändern und die physikochemische Eigenschaften des Peptids verbessern oder die kovalente Bindung des Peptids an einem festen Carrier, an Carrier-Protein oder an ein anderes Peptid erleichtern.

Darüber hinaus ist es auch möglich, durch den Austausch einzelner Aminosäuren durch strukturverwandte Aminosäuren immunreaktive Mutanten zu erzeugen. So kann beispielsweise die Aminosäure Val durch Leu, Ile oder nicht natürlich vorkommende Aminosäuren, wie Nva ersetzt werden.

Im allgemeinen führen solche Modifikationen nicht zu direkten Veränderungen der Immunreaktivität eines Peptides, allerdings können durchaus verbesserte immunologische Eigenschaften der Peptide erzielt werden. So neigt z. B. Methionin zu spontaner Oxidation, was durch Austausch gegen Norleucin verhindert werden kann, ohne daß sich die antigenen Eigenschaften des Polypeptides im wesentlichen ändern.

Weitere Aminosäurenaustausche können beispielsweise innerhalb folgender Gruppen vorgenommen werden: Gly, Ala; Val, Ile, Leu; Asp, Glu; Asn, Gln; Ser, Thr; Lys, Arg; Phe, Tyr; Ala, Ser; Ala, Thr; Ala, Val; Ala, Pro; Ala, Glu; Leu, Gln; Gly, Phe; Ile, Ser und Ile, Met.

Ein weiterer Gegenstand der Erfindung sind daher Peptide mit einer durch Ersatz, Hinzufügen oder Weglassen von einer oder mehreren Aminosäuren modifizierten erfindungsgemäßen Aminosäuresequenz.

Ebenfalls kann es vorteilhaft sein, in Form des Zusatzes einer hydrophoben Sequenz, umfassend etwa 2 bis 20 hydrophobe Aminosäuren wie z. B. Phe Ala Phe Ala Phe, die Adsorptionseigenschaften des Polypeptides an einen Träger zu verbessern.

Es wurde auch gefunden, daß für einen immunchemischen Anti HCMV-Nachweis Mischungen der erfindungsgemäßen Peptide bessere diagnostische Eigenschaften haben können als einzelne Peptide.

Ein weiterer Gegenstand der Erfindung sind daher Mischungen der erfindungsgemäßen Peptide.

Besonders geeignet sind Mischungen der Peptide 1,2 und 3, wobei wiederum besonders folgende Mischungen: 1.6, 2.1, 3.2; 1.11, 2.1, 3.2; 1.6, 2.10, 3.2; 1.11, 2.10, 3.2; 1.6, 2.7, 3.2 oder 1.11, 2.7, 3.2 bevorzugt sind.

Wird von den Peptiden jedoch eine Mischung hergestellt und damit beispielsweise eine Mikrotiterplatte beschichtet, so besteht die Gefahr einer ungleichen Beschichtung, da die Peptide aufgrund verschiedener physikalischer Eigenschaften unterschiedlich gut an die Oberfläche binden.

In einer weiteren Ausführungsform werden daher 2 bzw. mehrere der genannten Peptide, vorzugsweise 2 bis 10, inbesondere 2 bis 4 Peptide mit oder ohne Spacer verknüpft. Dadurch wird eine gleichmäßige Beschichtung der Mikrotiterplatten erreicht. Es können sogar polymere Formen zweier oder mehrerer Peptide nach den Fachmann bekannten Methoden hergestellt werden, so daß mehrere immunrelevante Epitope auf einem Peptid vorliegen. Die erfindungsgemäßen Peptide können, wie bereits erwähnt, auch an einen Carrier wie z. B. Protein oder Latexpartikel gebunden werden. So eignen sich besonders als Carrier aber auch als Brücke beispielsweise humanes Serumalbumin und/oder Polylysin.

Derartige Modifikationen verändern im allgemeinen die passive Adsorption oder kovalente Bindeeigenschaft an die Festphase positiv, beeinflussen das Kopplungsverfahren vorteilhaft oder wirken stärker als Antigen bei der Erzeugung von gegen die Peptide gerichtete polyklonale oder monoklonale Antikörper.

Gegenstand der Erfindung sind daher auch Peptide, die untereinander mit oder ohne Brücke verknüpft sind, beziehungsweise auch an einen Carrier gebunden sein können.

Die genannten immunoreaktiven Peptide können synthetisch oder gentechnologisch, vorzugsweise synthetisch nach dem Fachmann bekannten Verfahren hergestellt werden.

Sie können sowohl als ein Peptid, als auch als Mischung mehrerer kleiner Peptide mit überlappender oder nicht überlappender Aminosäure-Sequenz synthetisiert werden.

Unter die gentechnologisch hergestellten Polypeptide fallen auch Fusionsproteine, deren Fusionsanteil nachträglich abgespalten wurde. Auch fallen Polypeptide darunter, die gegebenenfalls beispielsweise durch Glykosylierung, Acetylierung oder Phosphorylierung modifiziert werden können.

Für die chemische Peptidsynthese wurde vorzugsweise die Festphasensynthese, insbesondere die Fmoc-Methode nach G. B. Fields und R. L. Noble benutzt. Nach dieser Methode können die erfindungsgemäßen Peptide beispielsweise an einem halb - oder vollautomatischen Peptidsynthesizer an Polystyrol (1 % Divinylbenzol) unter Verwendung Fmoc gängiger Anker hergestellt werden. C-terminal kann z. B. eine Carboxyl- oder eine Carboxamidfunktion verwendet werden. Im Falle einer Carboxylgruppe kann vorzugsweise als Anker Alkoxybenzylalkohol am Harz benutzt werden. Die Synthese von Peptidamiden kann beispielsweise an [(5-Carboxylatoethyl-2,4-dimethoxyphenyl)-4'-methoxyphenyl]-methylaminharz nach Breipohl et al.(G. Breipohl, J. Knolle und W. Stüber, Int. J. Peptide, Protein Res. 34, 1989, 252-267) ausgeführt werden. Im allgemeinen hatten die Harze Anfangsbeladungen im Bereich von 0,2 - 1,0 mmol, vorzugsweise 0,4-0,6 mmol Aminofunktionen/Gramm. Die Synthesen wurden nach folgender allgemeiner Reaktionsfolge durchgeführt:
1. Waschen des Harzes, beispielsweise mit DMF.
2. Behandlung des Harzes mit einem Lösemittelgemisch, wie z. B. Piperidin / DMF.
3. Waschen des Harzes, vorzugsweise zuerst mit DMF / Isopropanol und anschließend mit DMF.
4. Umsetzung des Harzes mit einem 1-6fachen, vorzugsweise 2-4fachen, insbesondere 2,5-3,5fachen Überschuß an aktiviertem Aminosäurederivat, z. B. einer Fmocaktivierten Aminosäure. Die Aktivierung des Aminosäurederivates erfolgte beispielsweise mit DIC/HOBt oder TBTU/DIPEA in DMF.
5. Wiederholen der Reaktionsschritte 3 und 4.

Nach Synthese des gewünschten Peptids wurden die Peptide vom Harz beispielsweise durch Behandlung mit 90 % TFA, 5 % Ethandithiol, 5 % Resorcin bei Raumtemperatur abgespalten. Das abgespaltene Peptid kann anschließend in Ether auskristallisiert werden und nach allgemeinen Verfahren, wie HPLC, Ionenaustauscherchromatographie oder Gelpermeation, gereinigt werden. Die Zusammensetzung der Peptide kann mittels Aminosäureanalyse und/oder Massenspektrometrie überprüft und die Reinheit beispielsweise mittels HPLC geprüft werden.

Die Herstellung verbrückter Peptide erfolgte ebenfalls nach allgemein bekannten Verfahren. Für die Verbrückung gibt es u. a. folgende Möglichkeiten:
A) direkte Amidbindung;
B) Verbrückung über ein Peptid mit 1 bis 10, vorzugsweise 1 bis 5, insbesondere 1 - 3 Aminosäuren;
C) Thioether oder Disulfidbrücke.

Die Synthese der mit einer Aminosäure oder einem kurzen Peptid verbrückten Peptide kann nach der obengenannten Methode durchgeführt werden. Als kurzes Peptid eignet sich beispielsweise ein Triglycin. Die Verbindung der beiden Peptide über einen sogenannten Spacer, insbesondere über einen hydrophoben Spacer, wirkt sich, wie bereits erwähnt, im allgemeinen günstig auf die Wechselwirkung mit der Mikrotiterplatte aus. Besonders bevorzugte Aminosäuren als Brückenglieder sind beispielsweise die epsilon-Aminocapronsäure.

Thioether oder Disulfidbrücken werden durch Synthese einer Maleimidfunktion an den N-Terminus des einen Peptids und Einbringung einer Sulfhydrylgruppe, vorzugsweise in Form von Cystein, an den C - oder N-Terminus des anderen Peptids, und anschließender Verknüpfung beider Peptide erhalten. Diese Verknüpfung kann sowohl an einem festen Träger als auch in Lösung erfolgen.

Unter einem immunchemischen Nachweis werden im allgemeinen Verfahren zusammengefaßt, die als homogene (in Lösung) oder heterogene (mit fester Phase) Methoden die Bestimmung von Antigenen und/oder Antikörpern in Körperflüssigkeiten wie Serum, Plasma, Speichel, Liquor oder Urin erlauben. Beispiele für diese auch sogenannten Immunoassays sind Enzymimmunoassay (ELISA oder EIA), Radioimmunoassay (RIA), Immunfluoreszenzassay (IFA), Radioimmunopräzipitationsassay (RIPA) oder Agar Gel-Diffusionsassay usw.

Diese zahlreichen, sehr verschiedenen Methoden unterscheiden sich in speziellen Ausführungsformen, in dem zur Detektion verwendeten Marker oder Meßprinzip (z. B. photometrisch, radiometrisch, visuell bzw. per Aggregations-, Streulicht- oder Präzipitations-Verhalten) und in den Festphasen. Dem Fachmann ist es bekannt, daß eine Trennung von gebundenen und freien Proben-Antikörpern oder -Antigen zwar weit verbreitet aber nicht unbedingt erforderlich ist, wie z. B. bei sogenannten homogenen Assays. Bevorzugt sind heterogene Immunoassays, insbesondere heterogene ELISA-Verfahren.

Bei dem Antikörper-Nachweis setzt ein immunchemisches Nachweisverfahren während des Ablaufes den Kontakt der Probe mit den beschriebenen Peptidsequenzen voraus, um in einem bestimmten Schritt des jeweiligen Verfahrens einen Antigen-Antikörperkomplex auszubilden oder bei Kompetitions- und Inhibitionstests die Bildung desselben durch Zusatz von geeigneten markierten Reagenzien zu verhindern.

Ein weiterer Gegenstand der Erfindung ist daher ein immunchemisches Verfahren zum Nachweis und/oder zur Bestimmung von HCMV Antikörper unter Verwendung der erfindungsgemäßen Peptide und ein Testbesteck dazu.

In den direkten Verfahren können die Antikörper mit festphasengebundenen Peptiden oder mit markierten Peptiden oder mit beiden in Kontakt gebracht werden, wobei es unerheblich ist, ob das zugrundeliegende Verfahren als 1-, 2- oder Mehr-Schritt-Methode auf dem Prinzip des 2. Antikörpertests oder dem immunometrischen Testaufbau (Doppel-Antigen-Sandwich) entweder mit gleichen oder unterschiedlichen Peptiden (bzw. Peptidmischungen) auf der Festphase und als Flüssigreagenz für die Detektion sowie in Verbindung mit spezifischen sogenannten Capture-Antikörpern (z. B. Anti IgM) oder Affinitätsreagenzien (z. B. Protein A) beruht.

Die Bindung der Peptide an die Festphase kann kovalent, adsorptiv oder mittels spezifischer Antikörper oder ähnlich affiner Methoden, z. B. über den Biotin/Avidin-Komplex stattfinden, vorzugsweise jedoch adsorptiv.

Als Trägermaterial für die Festphase sind Kunststoffe wie Polystyrol, Polyvinylchlorid, Polyamid und andere synthetische Polymere, natürliche Polymere wie Zellulose sowie derivatisierte natürliche Polymere wie Zelluloseacetat und Nitrozellulose als auch Glas, insbesondere als Glasfaser, geeignet. Bevorzugt ist als Trägermaterial Polystyrol.

Die Träger können die Form von Kugeln, Stäben, Röhrchen und Mikrotiterplatten haben oder in Form von Suspensionen wie z. B. Latexpartikel vorliegen. Flächenförmige Gebilde wie Streifenpapiere, Plättchen und Membranen sind ebenfalls geeignet. Die Oberfläche der Träger kann sowohl für wässrige Lösungen durchlässig als auch undurchlässig sein.

Bevorzugte Träger sind Kugeln, Röhrchen, Näpfchen, Mikropartikel, Streifenpapiere und Membranen. Besonders bevorzugte Träger sind Mikrotiterplatten, Latexpartikel, Polystyrol-Kugeln oder magnetisch anziehbare Teilchen.

Die Peptidkonzentration bei der Beschichtung des Trägers beträgt im allgemeinen ca. 0,01 - 20 µg/ml, vorzugsweise 0,01 - 10 µg/ml, besonders bevorzugt 2 - 10 µg/ml.

Besonders vorteilhaft ist die Verwendung von synthetisch hergestellten Polypeptiden, deren hohe Reinheit und starke Antigenität die Verwendung kleinster Mengen von beispielsweise 0,01 - 2,0 µg/ml, vorzugsweise 0,1 - 0,5 µg/ml gestattet. Die Bindekapazität des Trägers, insbesondere bei Verwendung von Polystyrol, ist im allgemeinen nicht gesättigt, so daß normalerweise mit mehreren verschiedenen Polypeptiden, insbesondere mit 2 - 5, vor allem mit 3 - 4 verschiedenen Polypeptiden, beschichtet werden kann, was von besonderem Vorteil ist.

Bei Verwendung der Peptide als markierte Derivate zur Detektion kommen alle dem Fachmann bekannten Kopplungstechniken in Frage. Auch mehrstufige Verfahren wie z. B. präformierte Peptid-Antikörper-Komplexe, in denen der Antikörper die Markierung trägt, oder hochaffine Systeme, wie z. B. Biotin/Avidin mit Markierung eines dieser Reaktionspartner, können angewendet werden.

Als Marker können beispielsweise radioaktive Isotope, fluoreszierende oder chemilumineszierende Farbstoffe verwendet werden. Auch Enzyme, die z. B. durch chromogene, luminogene oder fluorogene Substratsysteme oder durch nachgeschaltete Verstärkersysteme mit einem zweiten Enzym, das durch das erste aktiviert wird nachgewiesen werden, können als Marker verwendet werden.

Vorzugsweise werden als Marker Enzyme, insbesondere die alkalische Phosphatase und/oder die Meerrettich Peroxidase oder chemiluminogenic compounds, wie z. B. Acridinium-Ester verwendet.

Die Markierung erfolgt nach Verfahren, die für die genannten Marker im Stand der Technik beschrieben sind.

Im Falle der Markierung der Antikörper mit Peroxidase kann die Periodat Technik nach NAKANE et al., 1974, J. Histochem. Cytochem. 22, 1084 - 1090, angewendet werden oder ein Verfahren gemäß ISHIKAWA et al., 1983, J. Immunoassay 4, 209 - 327, in dem die Partner mit einem heterobifunktionellen Reagenz verknüpft werden.

Neben diesen Verfahren können die Peptide zur Sensibilisierung von geeigneten Oberflächen wie z. B. Latex oder Erythrozyten verwendet werden, um durch Peptidspezifische Antikörper induzierte, physikochemische Veränderungen wie z. B. Präzipitationen, Aggregation oder Lichtstreuung automatisch oder visuell zu messen. Es ist bekannt, daß die Peptide auch nicht derivatisiert zur Inhibition dieser Meßprinzipien wie auch der zuvor genannten Methoden eingesetzt werden können.

Für den Nachweis der Antigene können immundiagnostische Verfahren verwendet werden, die sich polyklonaler oder monoklonaler Antikörper bedienen, welche mit Hilfe der erfindungsgemäßen Peptide oder Derivaten davon hergestellt werden. Die für das Nachweisverfahren in Frage kommenden Ausführungsformen sind dem Fachmann bekannt und dadurch gekennzeichnet, daß in einem bestimmten Schritt Antikörper-Antigen-Komplexe gebildet werden oder die Komplexbildung in Kompetitionsverfahren durch Zusatz eines markierten Antigens inhibiert wird.

Ein weiterer Gegenstand der Erfindung ist daher ein immunchemisches Verfahren zum Nachweis und/oder Bestimmung von HCMV Antigen unter Verwendung von Antikörper gegen die erfindungsgemäßen Peptide und Testbesteck dazu.

Für die Etablierung eines Antigentests kommen als Festphasen, Marker oder Meßprinzip alle bei der entsprechenden Antikörper-Bestimmung erläuterten Möglichkeiten in Frage, wobei das Kompetitionsprinzip und die Doppelantikörper-Sandwich Technik als immunchemische Methode besonders bevorzugt sind. Dabei ist es unerheblich, ob die Verfahren als 1-, 2- oder 3-Schritt-Verfahren ausgelegt sind. So können Mehrschrittverfahren mit unmarkierten Nachweisantikörpern durchgeführt werden, die mit Hilfe eines weiteren dagegen gerichteten Antikörpers bestimmt werden, der entsprechend markiert ist. Für die Erzeugung der Antikörper ist es vorteilhaft, die Peptide derart zu modifizieren, daß deren immunogene Eigenschaft verbessert wird, wie dies z. B. durch Kopplung an natürliche Proteine wie z. B. an Serumproteine wie Gammaglobulin oder Serumalbumin, an Hämocyanine wie KLH = keyhole limpet hemocyanin oder Toxoide wie Diphtherie oder Tetanustoxoid möglich ist (B.S. Schaffhausen in Hybridoma Technologie in the Biosciences and Medicine, ed. T.A. Springer, Plenum Press NY, London, 1985).

Schließlich kann die vorliegende Erfindung auch angewendet werden bei Verwendung eines immundiagnostischen Elements, das die Festphase und in trockener Form einen Teil oder auch alle erforderlichen Reagenzien enthält, wobei auch hier die neuen Peptide entweder festphasenseitig oder im Detektionsreagenz oder in beiden enthalten sind und eine Antikörper-Bestimmung, einen Antigen-Nachweis oder Kombinationen mit anderen Analyten durchgeführt wird.

Zum Austesten der erfindungsgemäßen Peptide kann beispielsweise eine Mikrotitrationsplatte mit den Peptiden beschichtet werden. Die Beschichtung wird im allgemeinen in einer Pufferlösung, beispielsweise in einem Carbonat-, Phosphat- oder Trishydroxymethylaminomethanpuffer, vorzugsweise in einem Carbonatpuffer durchgeführt. Nach der Beschichtung wurden die Platten mit Serumproben in einer Verdünnung von 1:1 bis 1:250, vorzugsweise 1:1 bis 1:10, insbesondere 1:1,5 bis 1:2,5 inkubiert. Anschließend wurde der Antigen-Antikörper-Komplex nach den oben aufgeführten Methoden, beispielsweise mit einem markierten zweiten Antikörper gegen den Antigen-Antikörper-Komplex, nachgewiesen (Immunotest).

Ein weiterer Gegenstand der Erfindung ist ein immunchemisches Verfahren und ein Kombinationsbesteck dazu zum Nachweis und/oder zur Bestimmung von mehreren verschiedenen Antikörperspezifitäten gegen jeweils unterschiedliche Pathogene, wobei ein oder mehrere der erfindungsgemäßen Peptide von HCMV und ein oder mehrere immunreaktive Peptide von anderen Pathogenen an einen Träger fixiert werden und die Antikörper dagegen nach den oben genannten Verfahren differenzierend oder nicht-differenzierend, vorzugsweise differenzierend nachgewiesen werden.

In dem Kombinationstest können mit HCMV verwandte Virusarten, wie z. B. die Herpesviren Herpes simplex 1 und/oder 2 (HSV 1/2), Epstein-Barr-Virus (EBV), Varicella zoster Virus (VZV) oder Humanes Herpesvirus 6 (HHV 6) und/oder nicht verwandte Virusarten, wie z. B. die Hepatitisviren HAV, HBV, HCV oder auch die Viren HIV1 bzw. HIV2 eingesetzt werden. Insbesondere können Gemische von HCMV Peptiden und einem oder mehreren Peptiden und/oder rekombinanten Proteinen von anderen Pathogenen in dem Kombinationstest eingesetzt werden, wobei die Peptide untereinander keine meßbare Kreuzreaktivität besitzen sollten.

Die erfindungsgemäßen Peptide, deren immunreaktiven Teile und Mutanten sind beispielsweise für die HCMV-Diagnostik und Vakzinierung geeignet. Die Peptide sind auch als Beschichtungsantigene für unterschiedliche, diagnostische Testsysteme, die mit verschiedenen Oberflächen arbeiten, z. B. Enzymimmunoassay, Magnet- und Latexpartikel, Teststreifen, Folien und Papiere unterschiedlicher Herstellungsart usw. zu verwenden.

Ein Vorteil der erfindungsgemäßen immunreaktiven Peptide ist eine hohe Sensitivität und Spezifität zum Nachweis von HCMV-Antikörper, so daß neben verdünnten auch unverdünnte oder nur schwach verdünnte Proben verwendet werden können. Ein weiterer Vorteil der Peptide ist, daß sie aufgrund ihrer relativ kurzen Sequenzlänge einfach und in hoher Ausbeute chemisch synthetisiert werden können. Die chemische Synthese hat den Vorteil, daß die synthetischen Peptide frei von zellspezifischen Proteinen sind.

Ferner sind Gegenstand der Erfindung DNA Sequenzen, die für mindestens eines der erfindungsgemäßen Peptide codieren.

Gegenstand der Erfindung ist auch ein analytisches Verfahren zum Nachweis und/oder zur Bestimmung von HCMV, wobei als spezifischer Schritt eine Hybridisierungsreaktion eingesetzt wird, in der mindestens eine Nukleinsäuresonde verwendet wird, die in ihrem spezifischen Teil komplementär zu mindestens einer der erfindungsgemäßen DNA Sequenzen ist.

Ein weiterer Gegenstand der Erfindung sind polyklonale und/oder monoklonale Antikörper, die gegen eines oder mehrere der erfindungsgemäßen Peptide gerichtet sind. Die Antikörper können durch Immunisieren eines geeigneten Spenders, beispielsweise eines Kaninchens, mit den erfindungsgemäßen Peptiden nach allgemein bekannten Methoden gewonnen werden.

Ein weiterer Gegenstand der Erfindung sind Vakzine gegen HCMV, die mindestens eines der erfindungsgemäßen Peptide oder Antikörper dagegen enthalten.

Ein weiterer Gegenstand der Erfindung ist ein Immunotest, der mindestens eines der erfindungsgemäßen Peptide enthält, insbesondere ein heterogener Immunotest.

### Abkürzungsverzeichnis

- DIC: Diisopropylcarbodiimid
- DIPEA: Diisopropyläthylamin
- DMF: Dimethylformamid
- Bop: Benzotriazol-1-yl-oxy-tris(dimethylamino)phosphoniumhexafluorphosphat
- Fmoc: Fluorenylmethoxycarbonyl
- HOBt: Hydroxybenzotriazol
- TBTU: 2-(lH-benzotriazol-1-yl)-1,1,3,3-tetramethyluroniumtetrafluoroborat

Die folgenden Beispiele sollen die Erfindung näher erläutern, ohne sie jedoch darauf zu beschränken:

### Beispiele

### 1. Herstellung von gly ala gly val asn val pro ala gly ala gly ala ala ile leu thr pro thr pro val asn pro ser thr ala pro ala pro ala pro-amid (Peptid 1.11)

1,06 g Fmoc-Amidankerharz wurden an einem vollautomatischen Peptidsynthesizer (Advanced ChemTech^{R}, Louisville, Kentucky, USA) nach Vorschrift des Herstellers umgesetzt. Der Aminosäureüberschuß war 3-fach. Die Reaktionszeit pro Aminosäurederivat betrug 30 Minuten. Als erste Aminosäure wurde 506 mg Fmoc-Prolin zusammen mit 213 mg HOBt und 697 mg Bop (anstelle TBTU) eingesetzt. Als Base wurde 3 mMol Diisopropylethylamin zugesetzt. Die Carboxamidfunktion war mit Trityl derivatisiert. Die Synthese wurde nach folgender Reaktionsfolge durchgeführt (für 1 g Harz):
1 Harz 3 mal mit je 15 ml DMF waschen
2 Harz mit 15 ml 20 % Piperidin/DMF behandeln (3 min)
3 Harz mit 15 ml 20 % Piperidin/DMF behandeln (10 min)
4 Harz 3 mal mit DMF/Isopropanol alternierend waschen
5 Harz 2 mal mit DMF waschen
6 Aminosäurederivat in 3-fachem Überschuß vorlegen und entweder mit DIC/HOBt oder mit TBTU/DIPEA in DMF aktivieren und 30 min mit dem Harz schütteln
7 Harz 2 mal mit DMF/Isopropanol alternierend waschen

Am Ende der Kopplungsreaktionen wurde das Peptidharz mit Methanol und Diethylether gewaschen und im Hochvakuum getrocknet. 2,36 Gramm Peptidharz wurden mit 27 ml Trifluoressigsäure/1,5 Gramm Resorcin/1,5 ml Ethandithiol 1,5 Stunden bei Raumtemperatur gerührt und in Diethylether kristallisiert. Das Rohpeptid wurde mit Diethylether gewaschen und im Hochvakuum getrocknet (Ausbeute 1090 mg). Das Peptid wurde an Sephadex G25 in 0,5 % Essigsäure chromatographiert und die Peptidfraktion durch Gefriertrocknung isoliert (Ausbeute 654 mg). Die Reinheit der Substanz wurde mittels HPLC geprüft. Die korrekte Zusammensetzung wurde durch Aminosäureanalyse bestätigt. Der Peptidgehalt betrug 86 %.

### 2. Austesten der Peptide

Eine Mikrotitrationsplatte mit 96 Kavitäten wurde mit einem synthetisierten Peptid beschichtet. Dazu wurden eine Lösung von 2,5 µg Peptid pro ml Carbonat-Puffer angesetzt und zu je 100 µl zur Beschichtung in die Kavitäten ausgebracht. Die Peptide wurden über Nacht bei 4°C an die Oberflächen adsorbiert und die Kavitäten nach Entfernung der Peptidlösung mehrfach mit Waschpuffer (50 mM Tris, pH 7,2, 10 mM EDTA, 0,2 % Tween 20) gewaschen. Danach wurden individuelle humane Serumproben in einer Verdünnung von 1:2 ausgebracht und 1 hr bei 37°C inkubiert. Nach einem weiteren Waschvorgang wurde der für humane IgG- bzw. humane IgM-Antikörper spezifische Konjugatantikörper, markiert mit Peroxidase dazugegeben und der Ansatz 0,5 hr bei Raumtemperatur inkubiert. Anschließend wurde die Reaktion durch Zugabe von 0,5 M Schwefelsäure gestoppt und die Extinktion der einzelnen Proben in einem Photometer bei einer Wellenlänge von 450/650 nm gemessen.

### 3. Lokalisierung relevanter Epitope

Zur Prüfung der Immunogenität wurden Peptide unterschiedlicher Länge aus dem gesamten Sequenzbereich Aminosäure 718 bis 880 des HCMV-Proteins pp150 (Tab. 2 und 3) wie in Beispiel 1 beschrieben, synthetisiert. Anschließend wurden nach Beispiel 2 die Mikrotitrationsplatten mit den synthetisierten Peptiden beschichtet und die immunologische Reaktivität getestet. Das Ergebnis der Testreihe war, daß alle geprüften HCMV-positiven Seren insbesondere mit mindestens einem der drei spezifischen Sequenzbereichen reagierten, wohingegen die übrigen Sequenzbereiche nur eine schwache immunologische Reaktion zeigten (Tab. 2 und 3). Die drei immunologisch reaktiven Peptide haben die Sequenzen: ASₑ-gly ala gly ala ala ile leu-BSₘ     (Peptid 1) CS_n-arg ala trp ala leu-DS_o (Peptid 2) und/oder ES_p-ala ser arg asp ala ala-FS_q (Peptid 3), wobei AS, BS, CS, DS, ES und FS unabhängig voneinander eine beliebige Aminosäure bedeuten und
e unabhängig voneinander ganze Zahlen von 0 bis 22,
m " " " " " 0 bis 25,
n und o " " " " " 0 bis 18 und
p und q " " " " " 0 bis 11 sind.

Hierbei konnten Seren differenziert werden, die sowohl mit allen, aber auch nur mit einem oder zwei Sequenzbereichen reagierten.

Nachweislich HCMV-negative Seren reagierten nicht mit den drei Peptiden.

### 4. Diagnostische Verwendung der erfindungsgemäßen Peptide

Um die Immunreaktivität der gefundenen Peptide zu prüfen, wurde ein Gemisch aus den Peptiden 1.11, 2.10 und 3.2 - Gesamtkonzentration 2,5 µg/ml bei einem Mischungsverhältnis von 1:1:1 (Einwaage) - entsprechend Beispiel 2 als Antigen auf Mikrotiterplatten ausgebracht und der Test wie dort beschrieben durchgeführt. Hierbei wurde ein definiertes und vorgeprüftes Serumkollektiv verwendet, das bereits in einem konventionellen Test auf IgM bzw. IgG-Reaktivität getestet worden war; Referenztests: CMV-Enzygnost^{R} IgM-POD bzw. CMV-Enzygnost^{R} IgG-POD, Behringwerke AG, Marburg.
Sowohl für den IgM als auch den IgG-Antikörpernachweis ist die angegebene Testkonfiguration sehr gut für die Diagnose des HCMV-Immunstatus geeignet (Tab. 4 und 5).

### 5. Reaktion des monoklonalen Antikörpers 87-55/02 mit einem der identifizierten Peptidepitope

Ein monoklonaler Antikörper, der eine eindeutige immunologische Reaktion mit dem HCMV-Protein pp150 als auch eine mit dem rekombinanten Proteinabschnitt pXP1 gemessen als Immunoblot- und ELISA-Reaktivität besitzt, wurde auf einer ELISA-Mikrotiterplatte untersucht, die mit den verschiedenen synthetisierten Peptiden der Tabelle 3 bei gleicher Konzentration (0,25 µg/Kavität) beschichtet worden waren. Nach Entwicklung der Platte mit genanntem Antikörper zeigte sich eine sehr scharf abgehobene Reaktion mit dem Peptid 2.7, das zuvor als ein wichtiges Epitop identifiziert worden war. Aus dem Selektionsverfahren des monoklonalen Antikörpers heraus wurde festgestellt, daß das identifizierte Epitop eine sehr wichtige Struktur besitzten muß, die auch durch Denaturierung des Gesamtproteins nicht zerstört wird. Dieser monoklonale Antikörper reagiert übereinstimmend sowohl mit dem synthetisierten Peptid 2.7 wie auch mit dem rekombinanten Protein XP1 und dem pp150-Protein des HCMV.

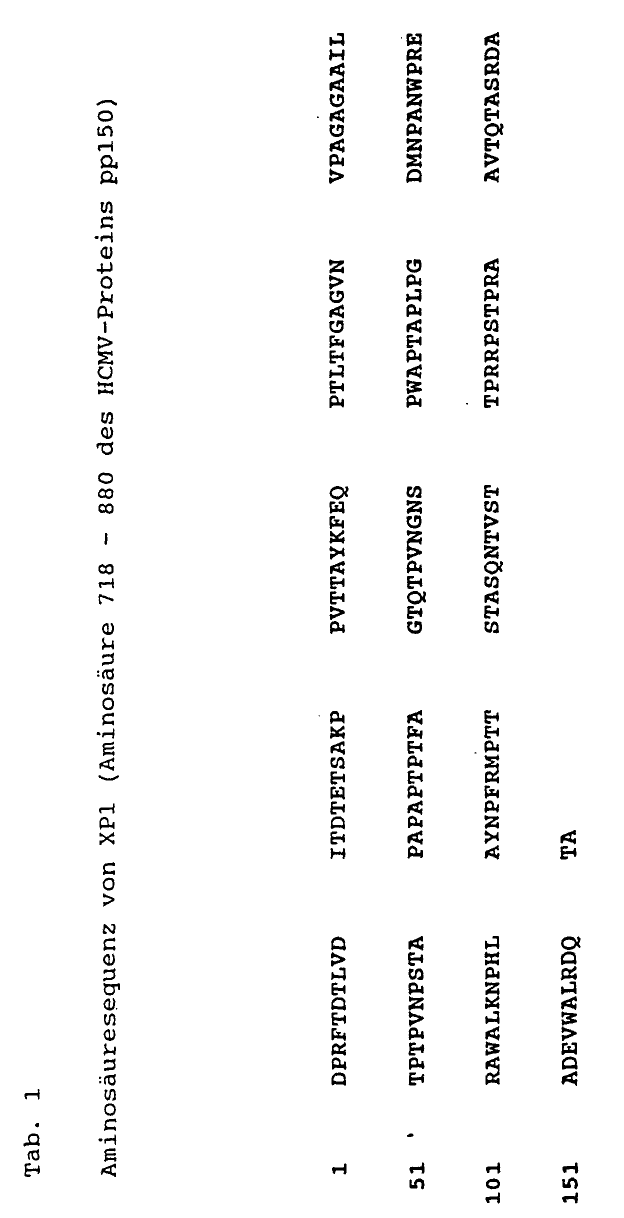

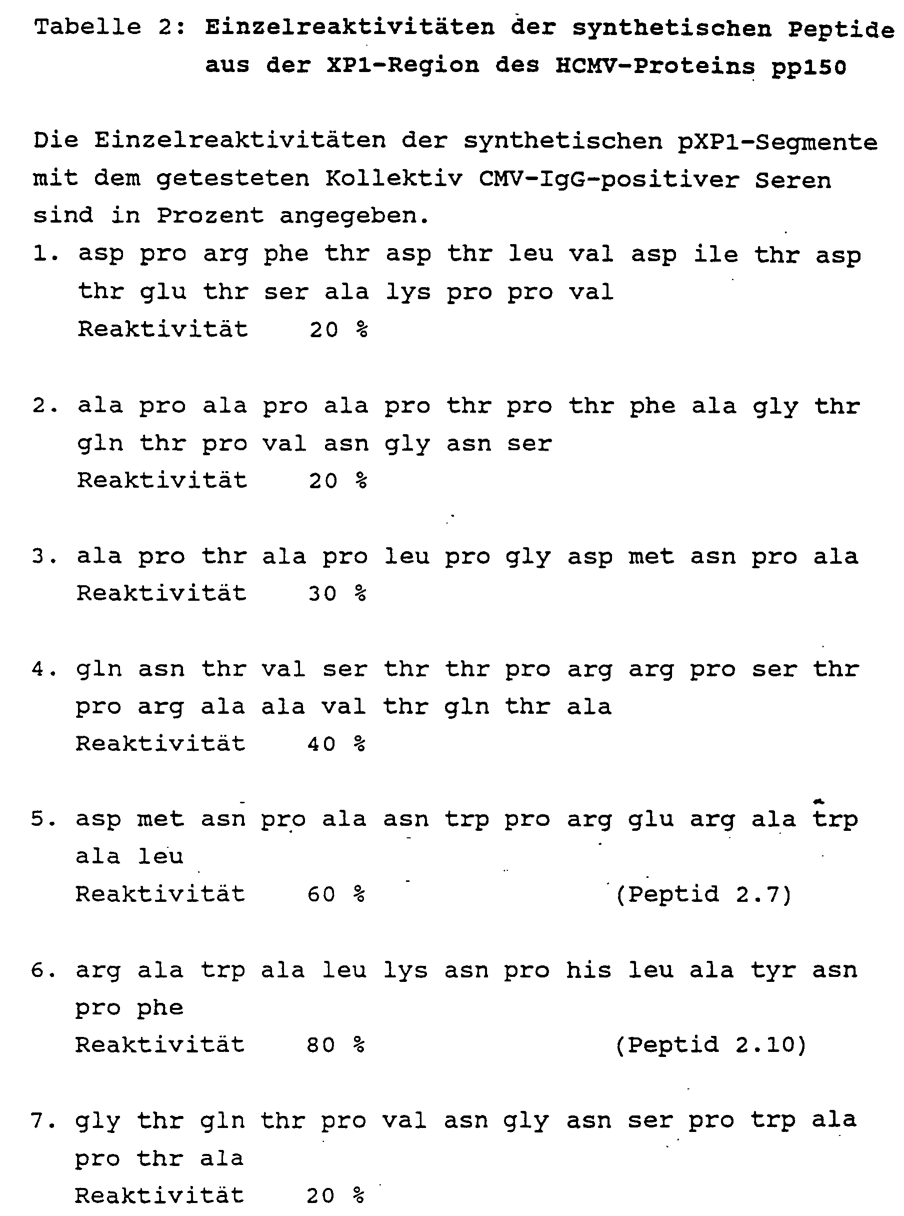

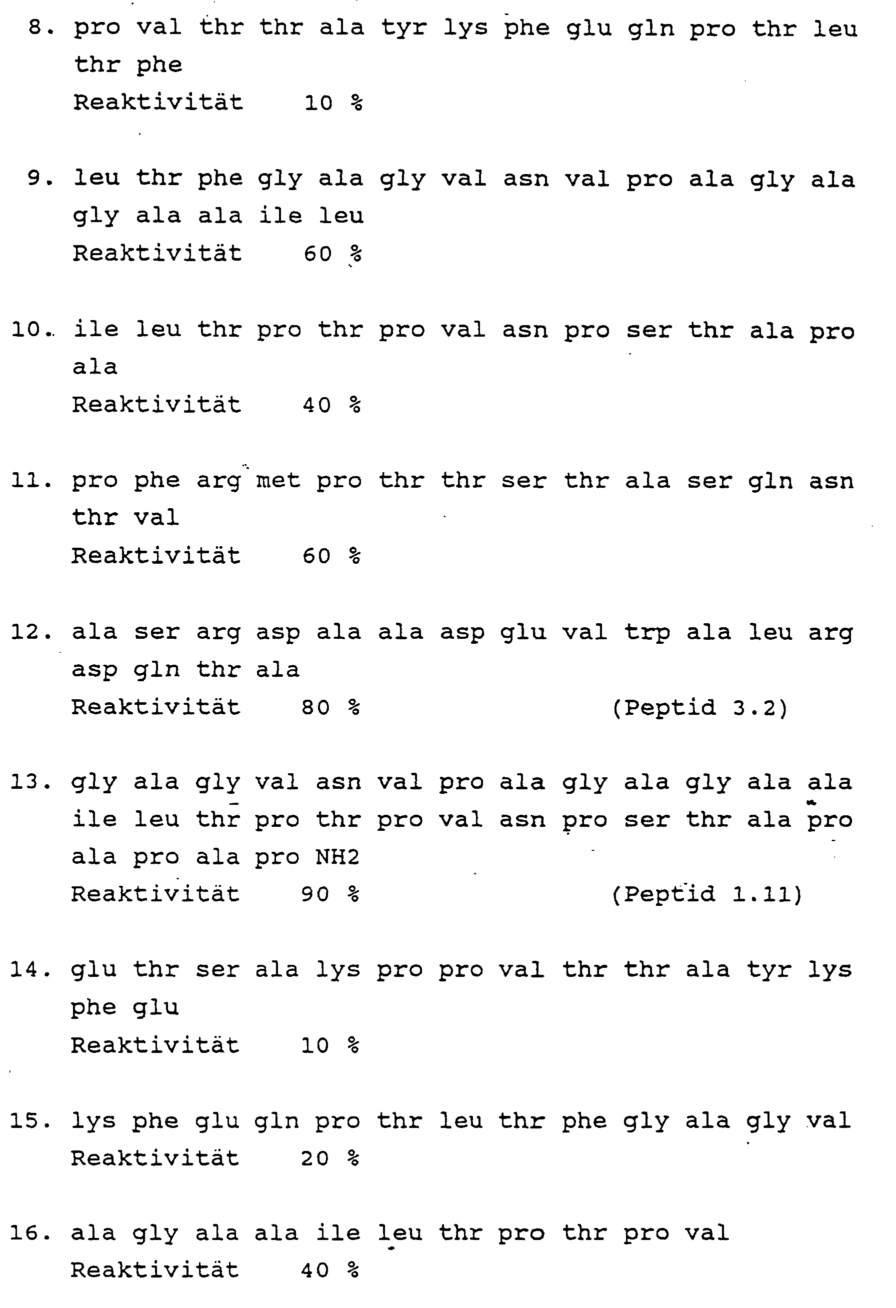

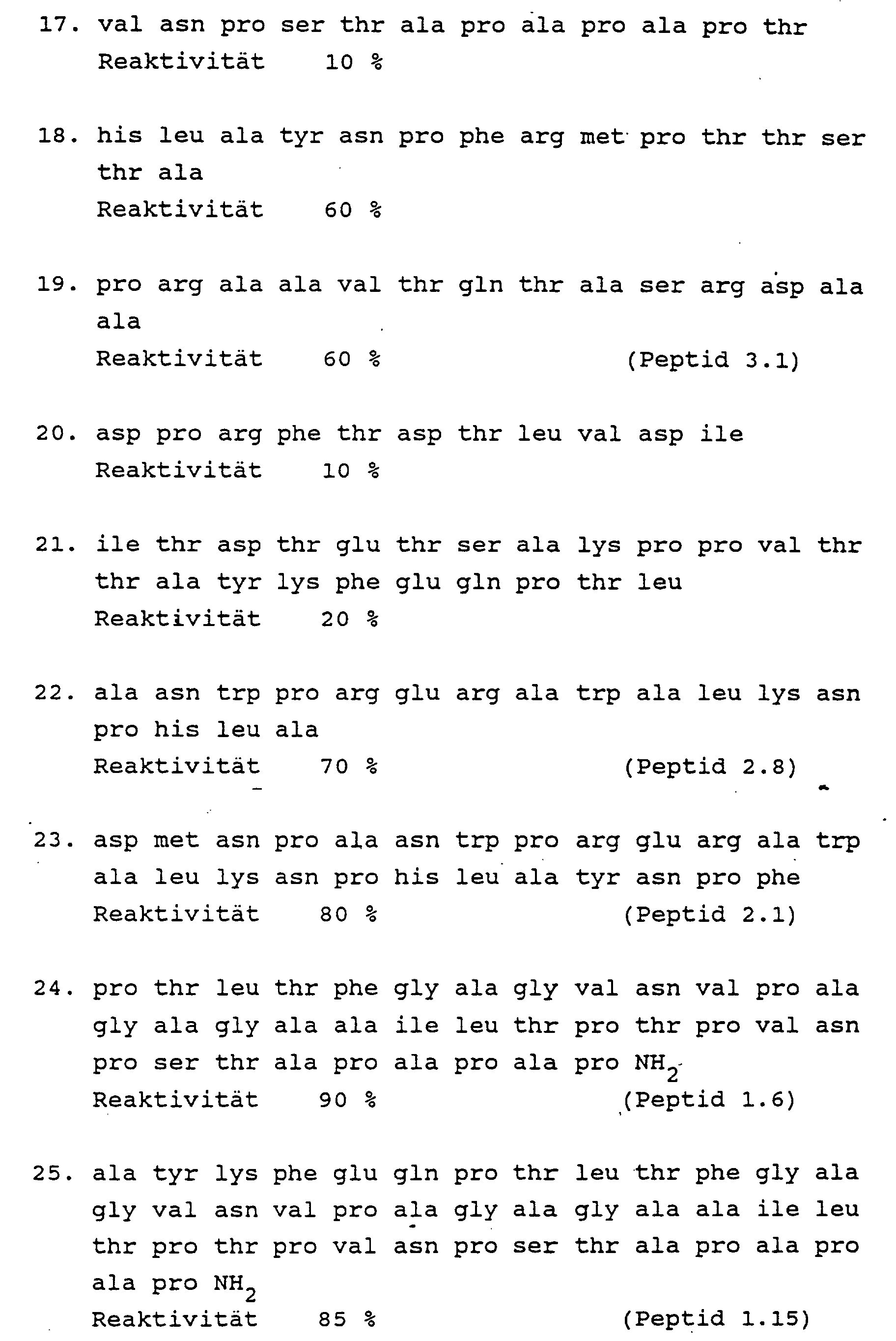

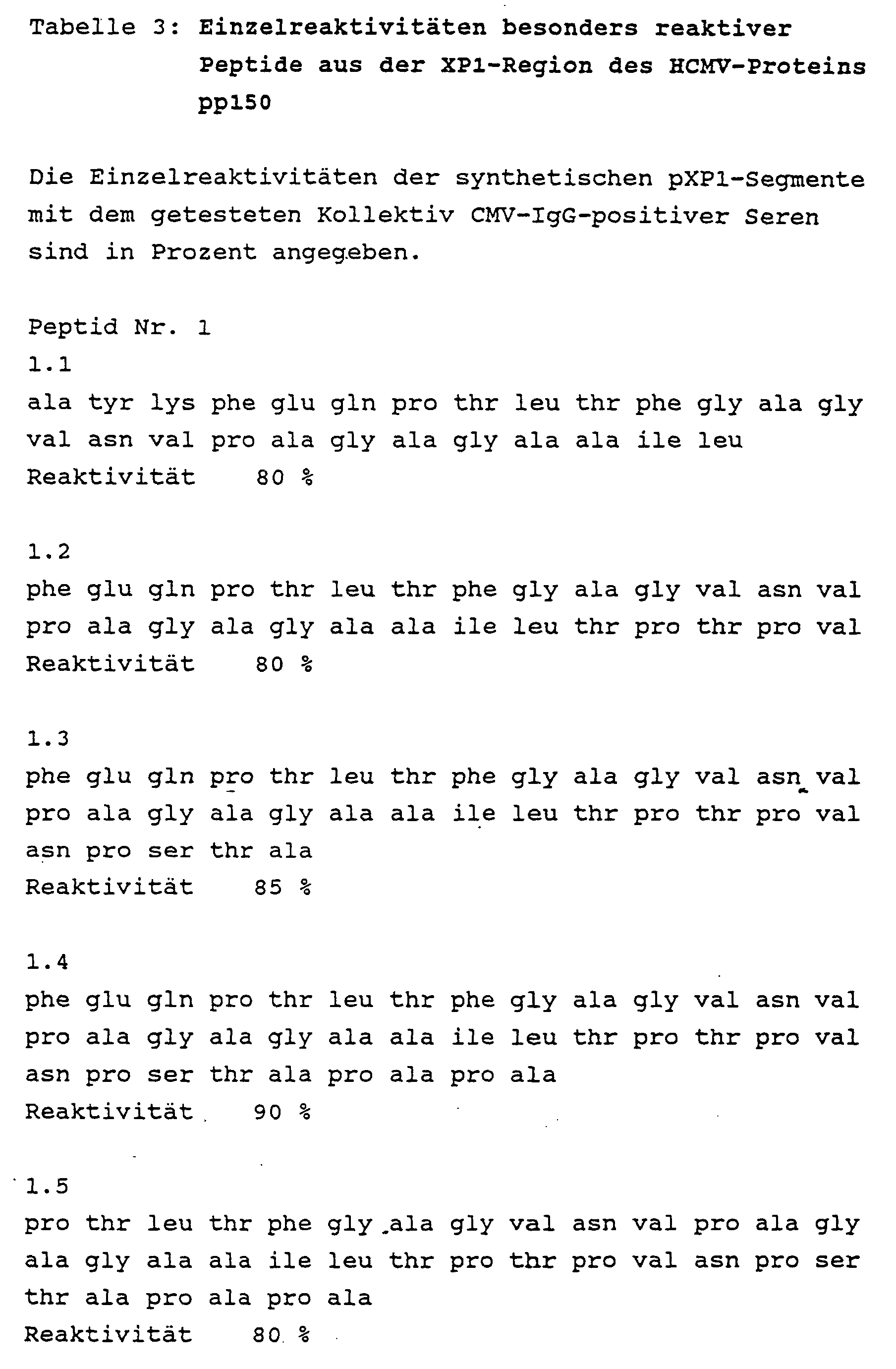

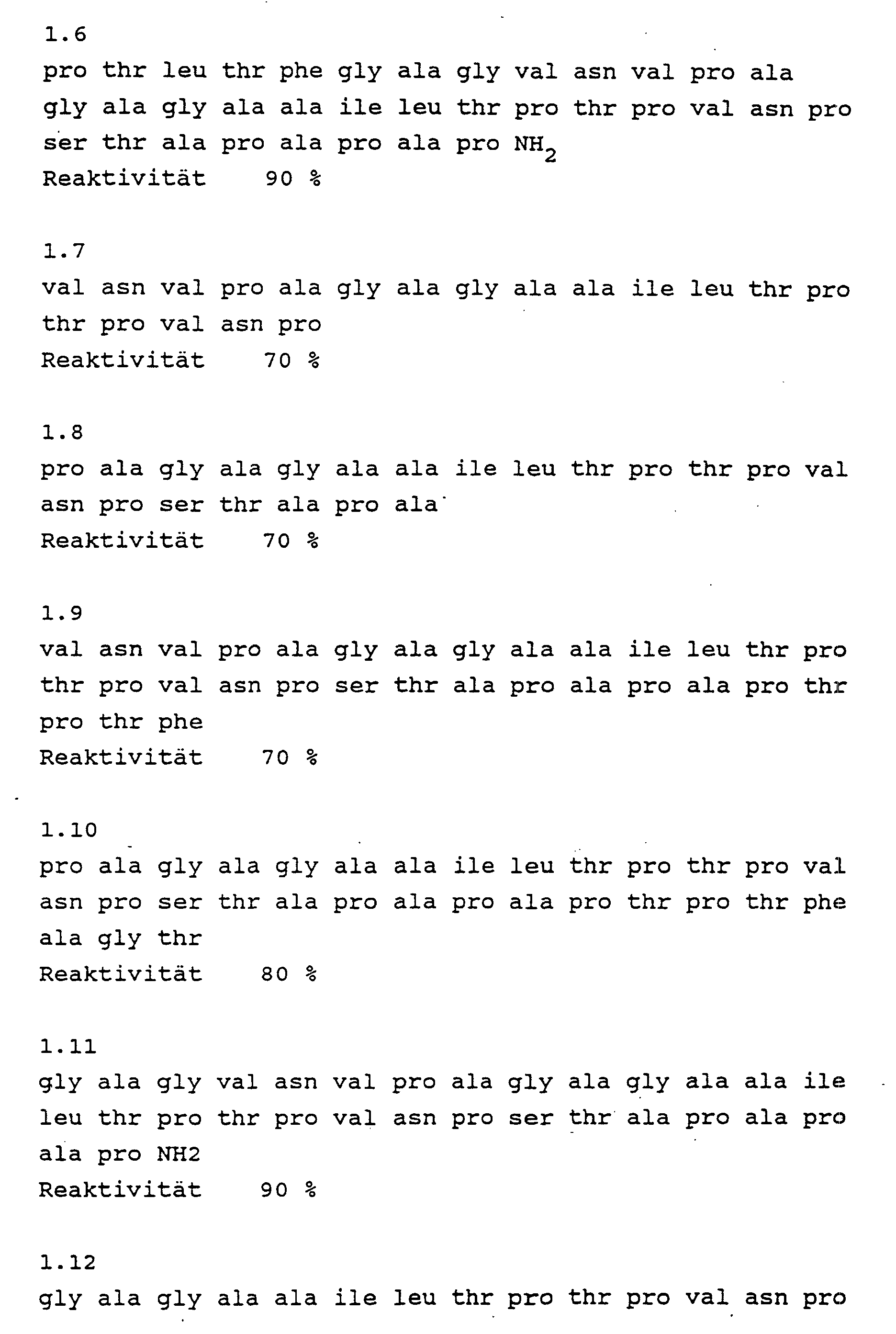

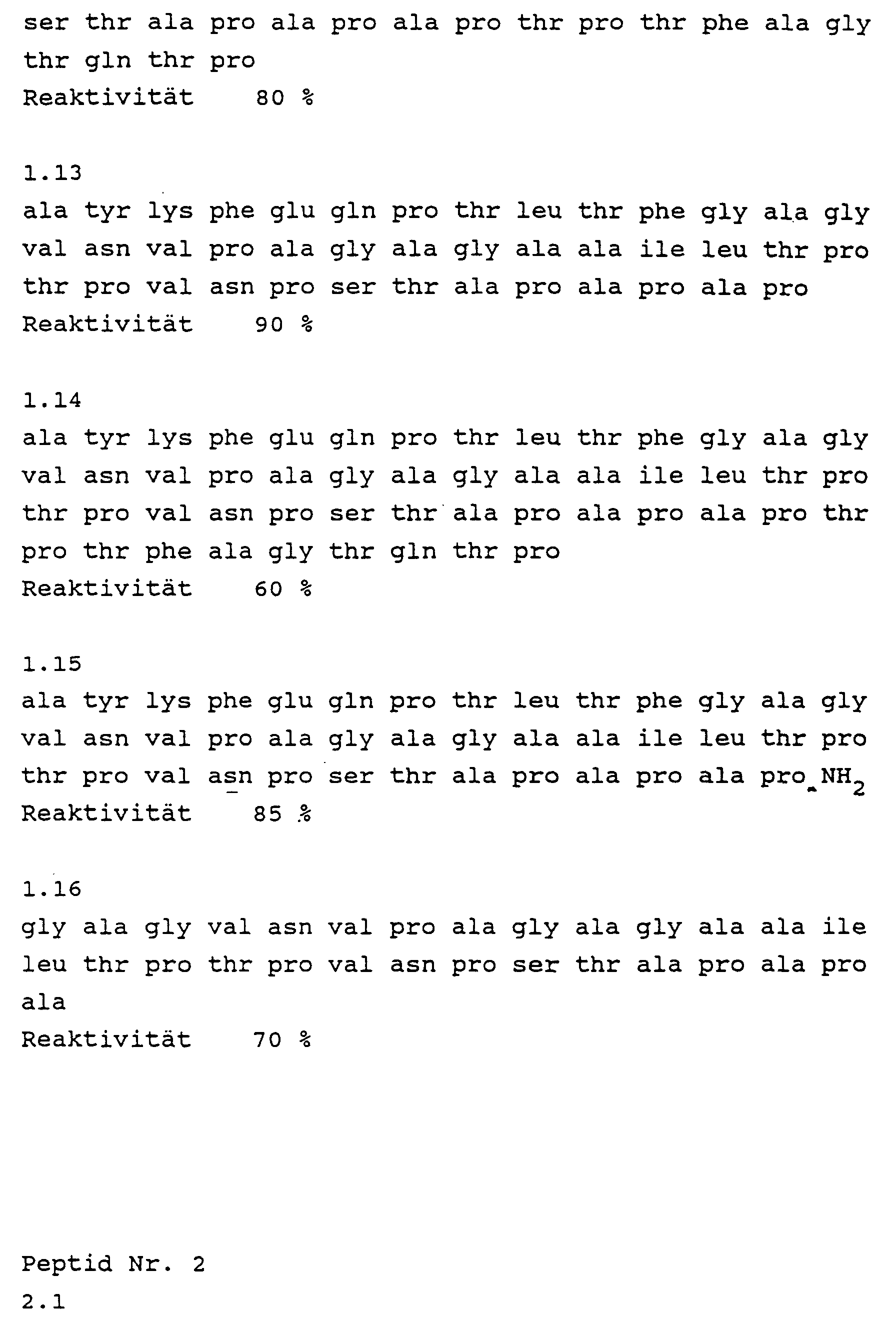

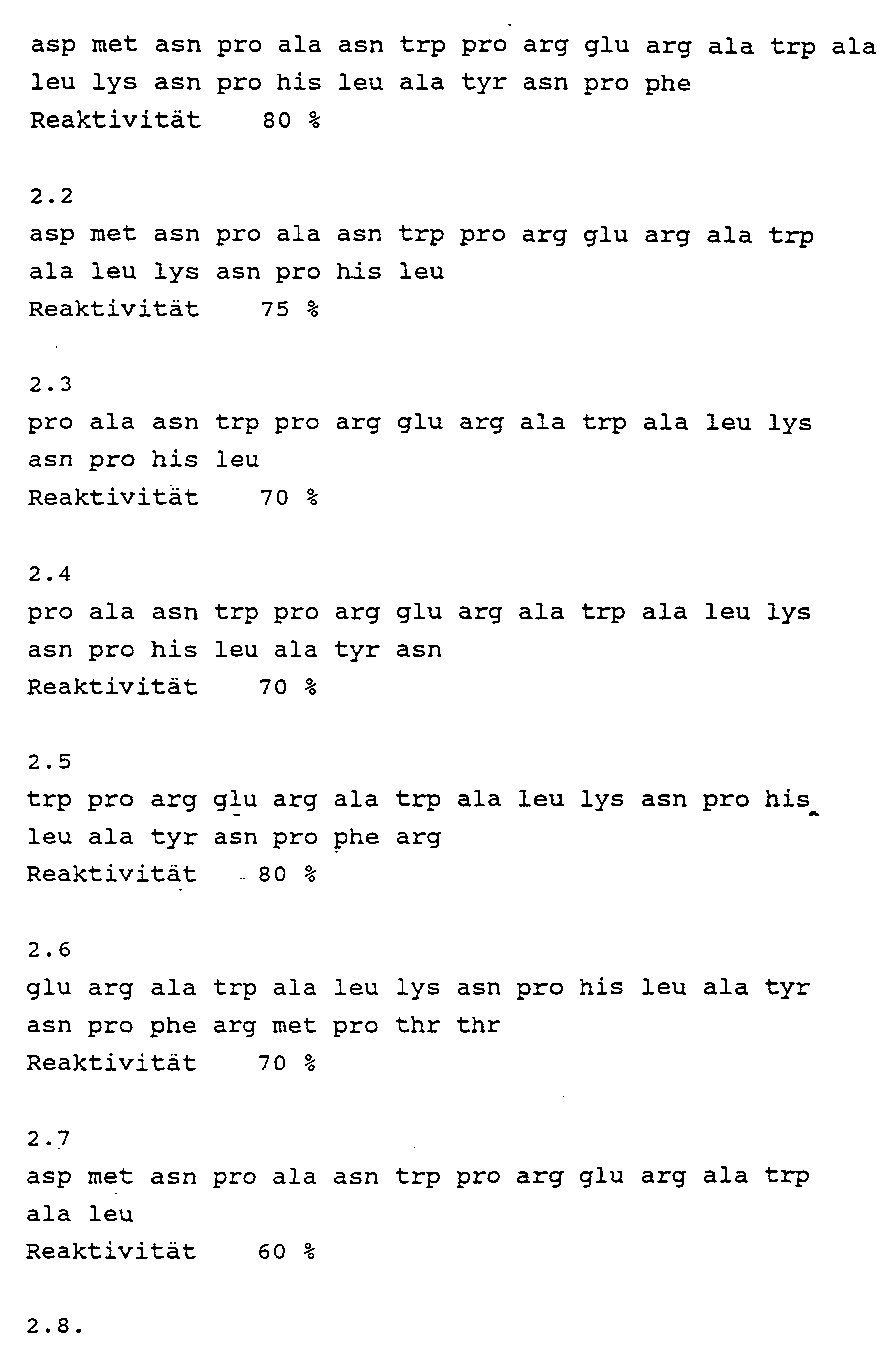

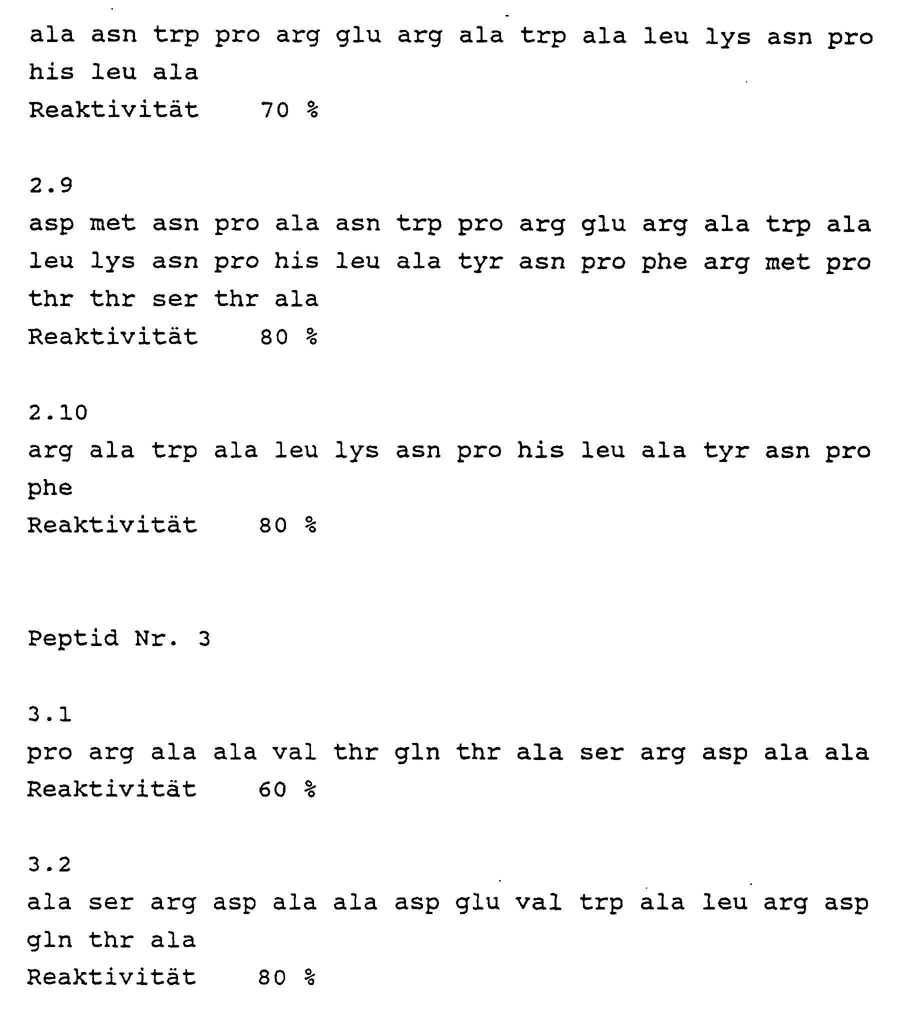

**Tab. 4:**

| **IgM-Reaktivität der Peptidmischung** | | |
|---|---|---|
| Bewertung | Referenztest | Peptidtest |
| positiv | 22 | 21 |
| negativ | 13 | 13 |
| falsch positiv | 0 | 0 |
| falsch negativ | 0 | 1 |
| | | |
| Anzahl getesteter IgM-Seren: | | 35 |
| Sensitivität : | | 95,5 % |
| Spezifität : | | 100 % |

**Tab. 5:**

| **IgG-Reaktivität der Peptidmischung** | | |
|---|---|---|
| Bewertung | Referenztest | Peptidtest |
| positiv | 133 | 132 |
| negativ | 152 | 149 |
| falsch positiv | 0 | 3 |
| falsch negativ | 0 | 1 |
| | | |
| Anzahl getesteter IgG-Seren: | | 285 |
| Sensitivität : | | 99,25 % |
| Spezifität : | | 98,03 % |

### Behringwerke AG - HOE 91/B 026 - Ma 886

### Sequenzprotokoll

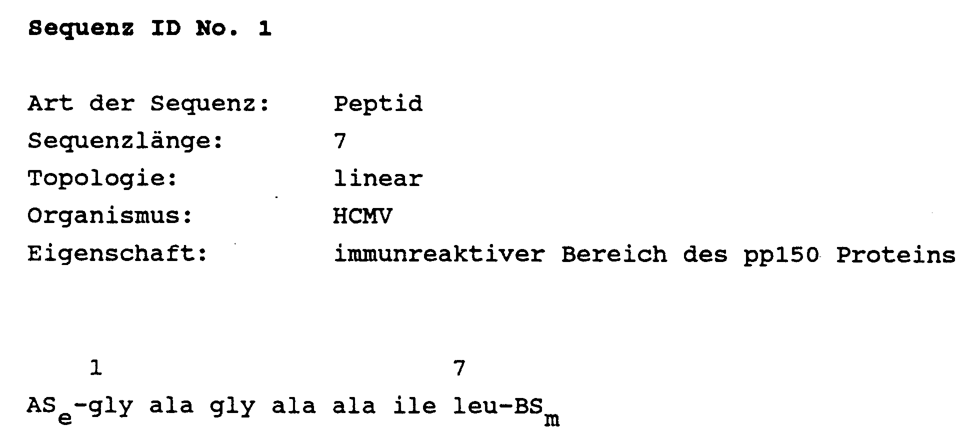

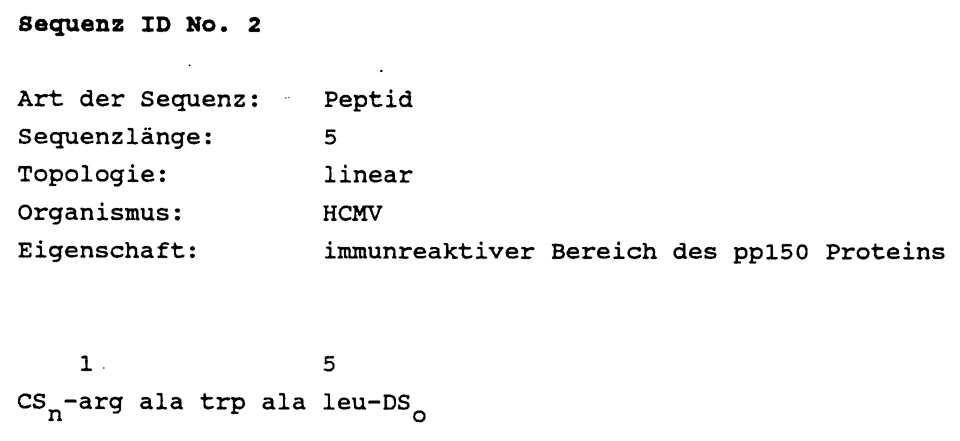

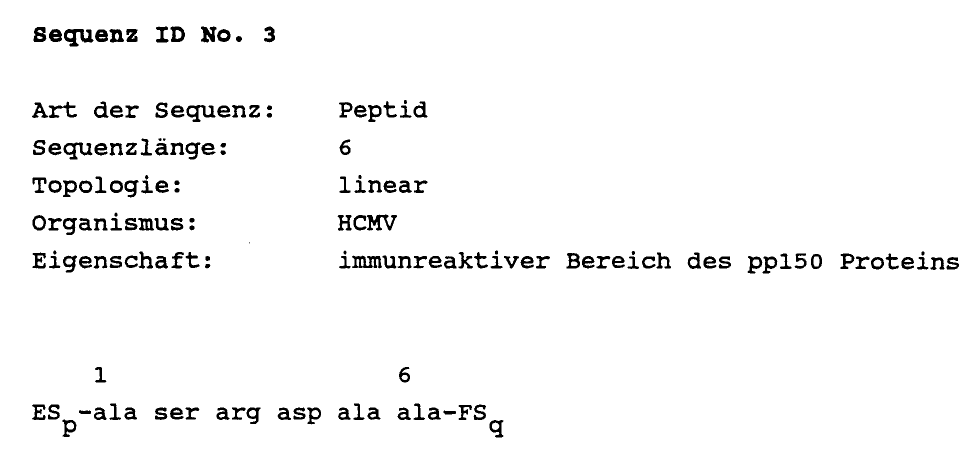

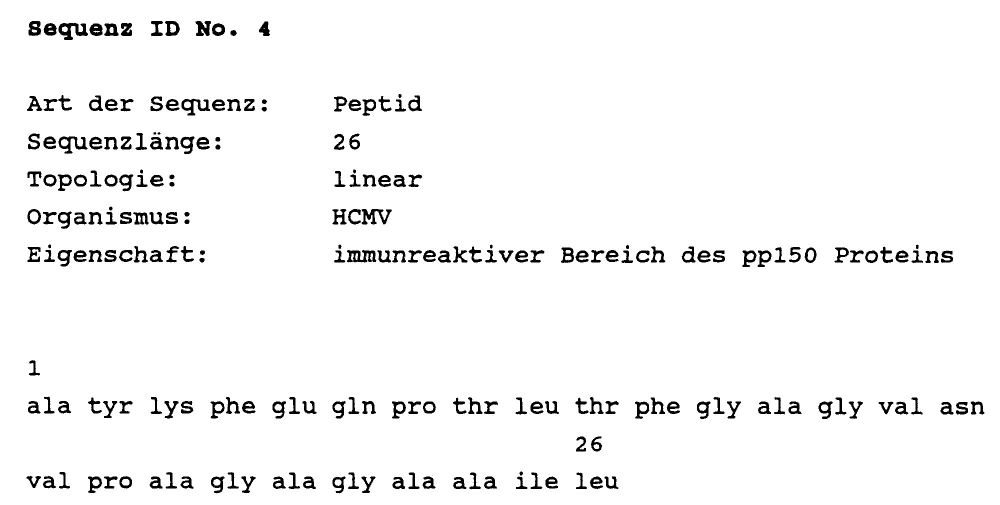

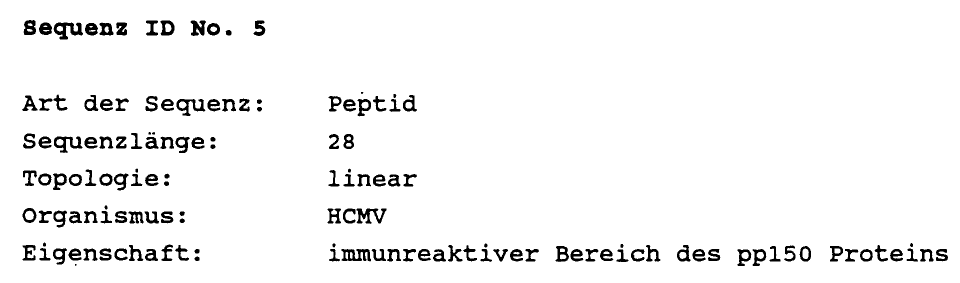

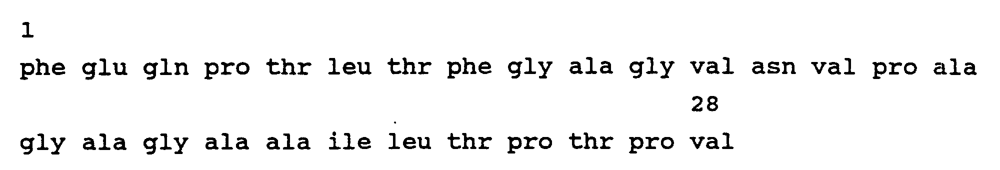

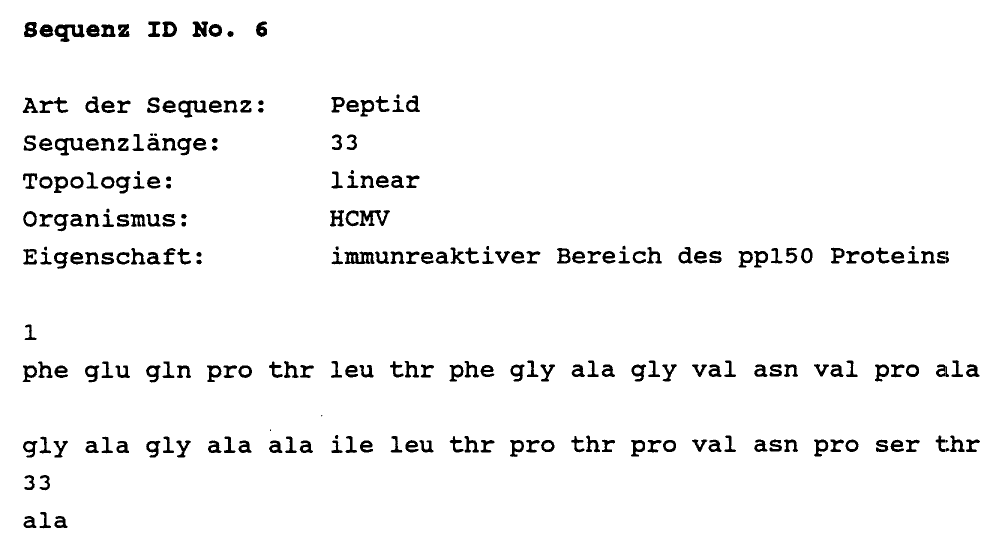

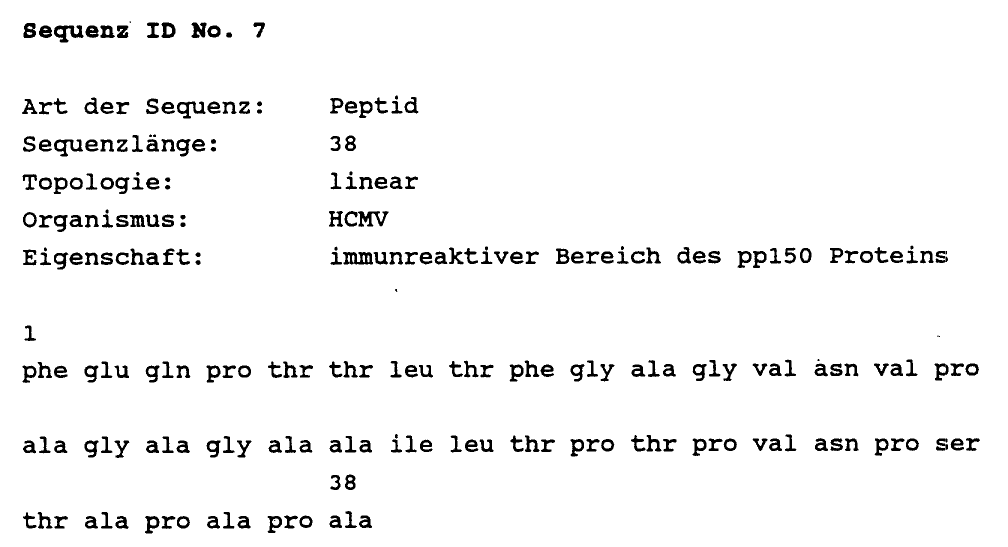

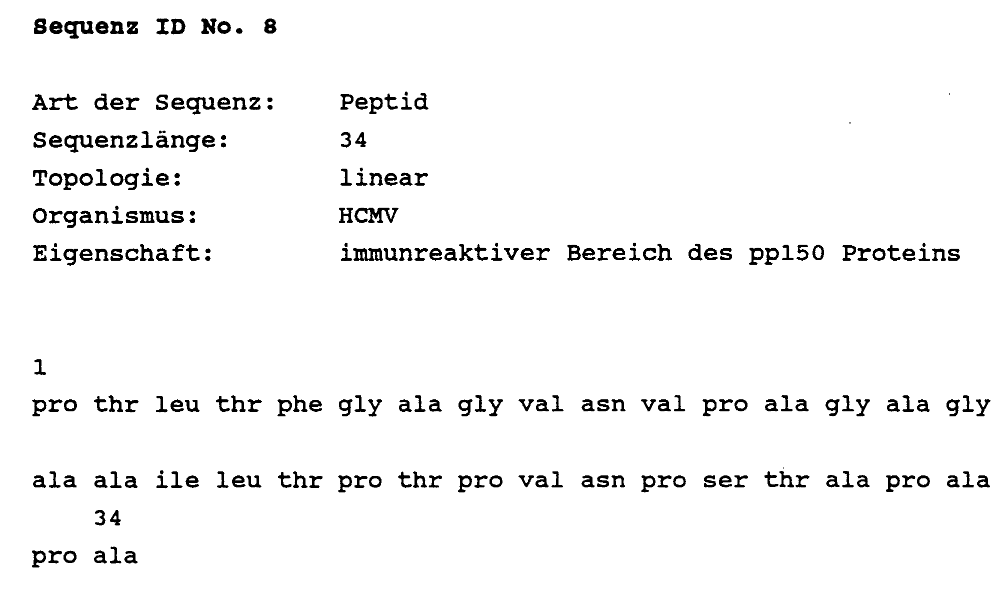

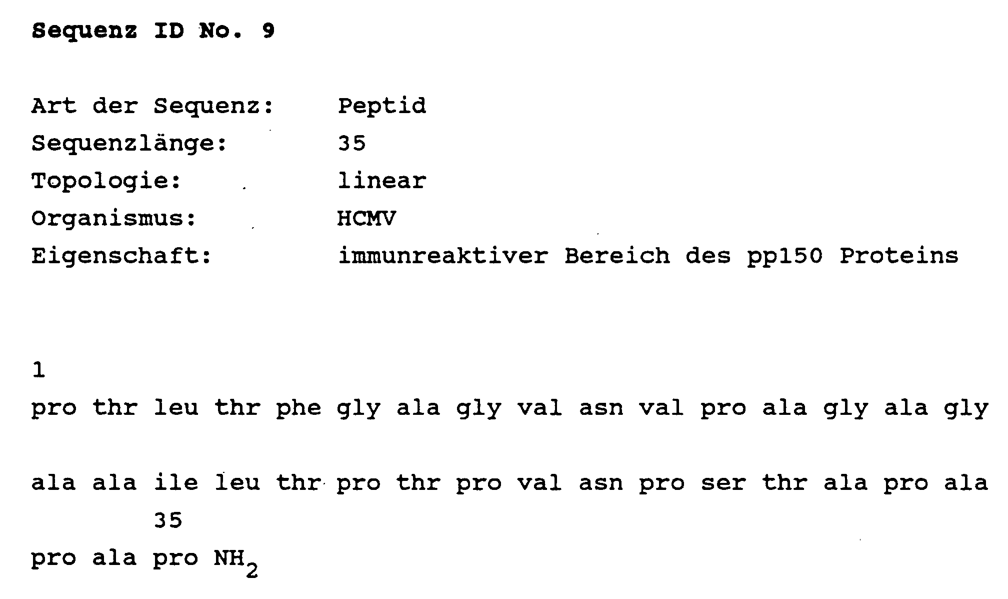

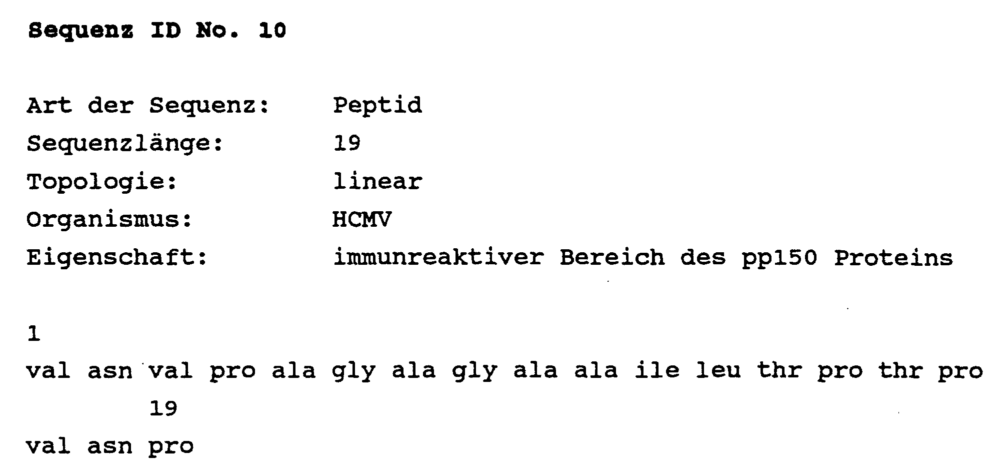

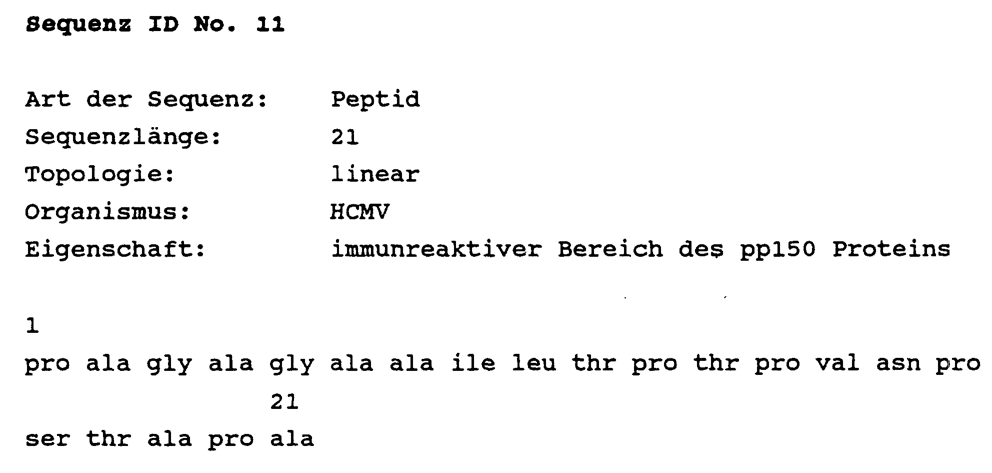

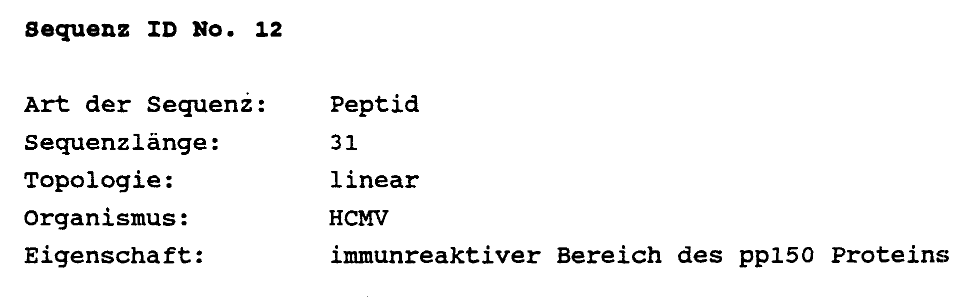

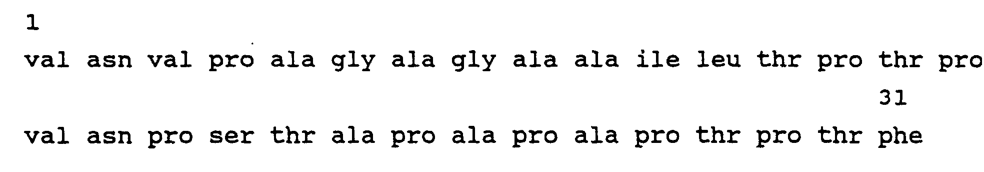

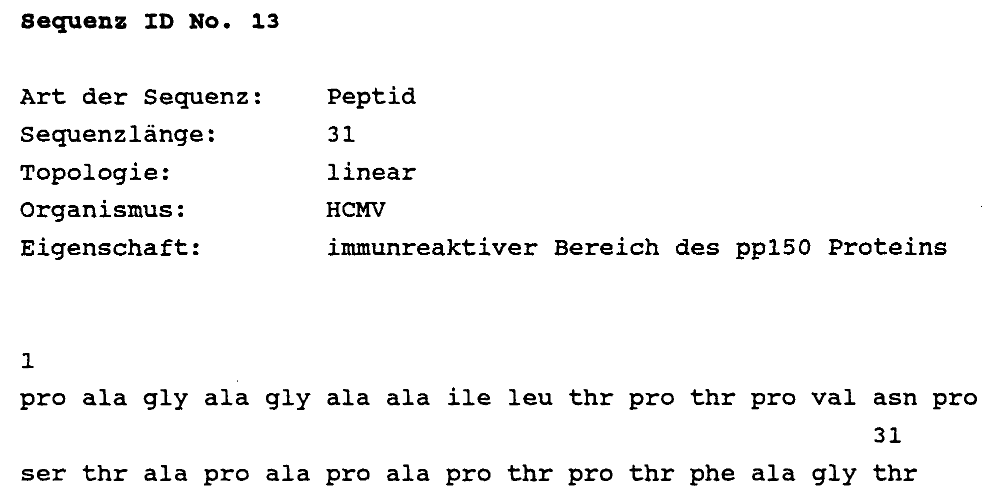

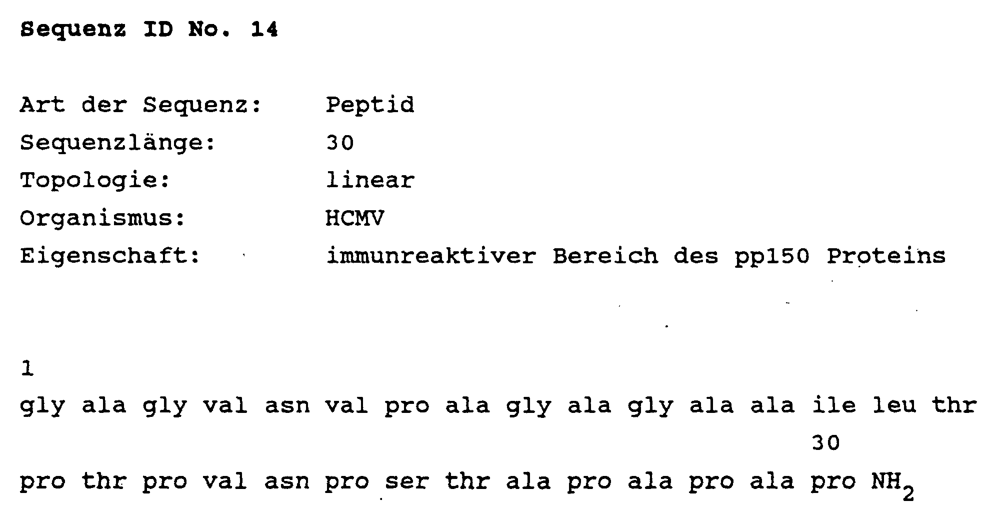

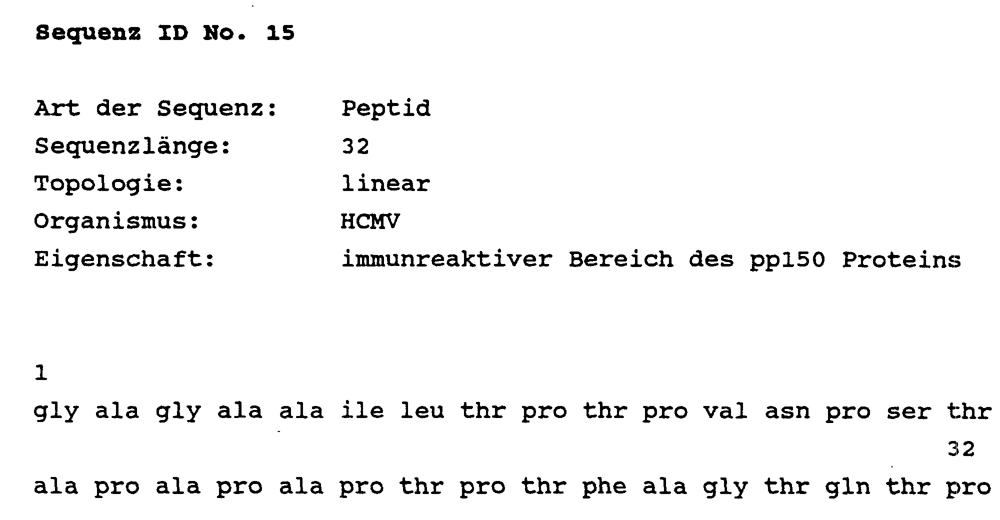

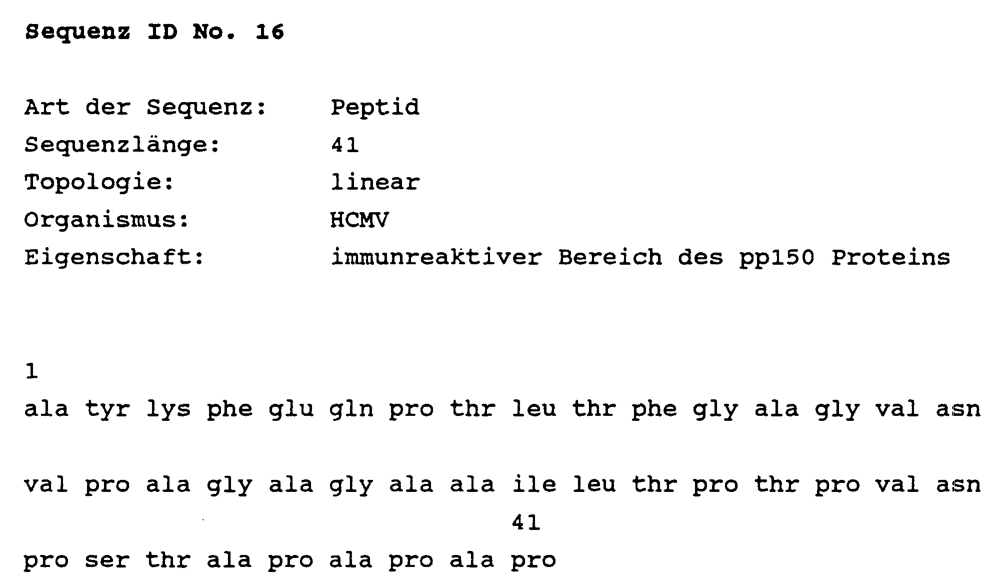

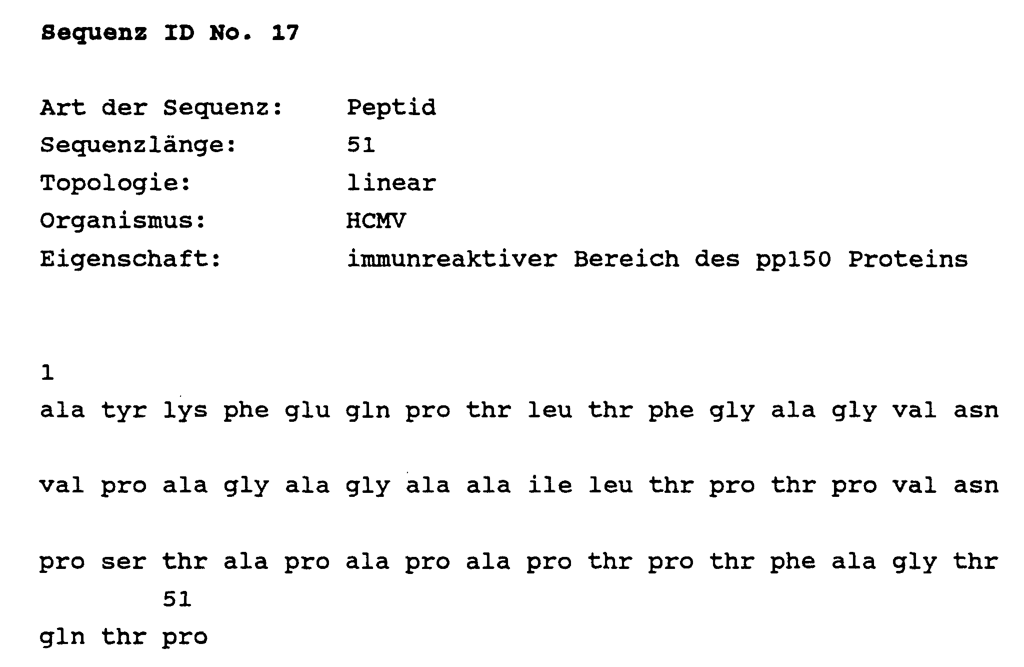

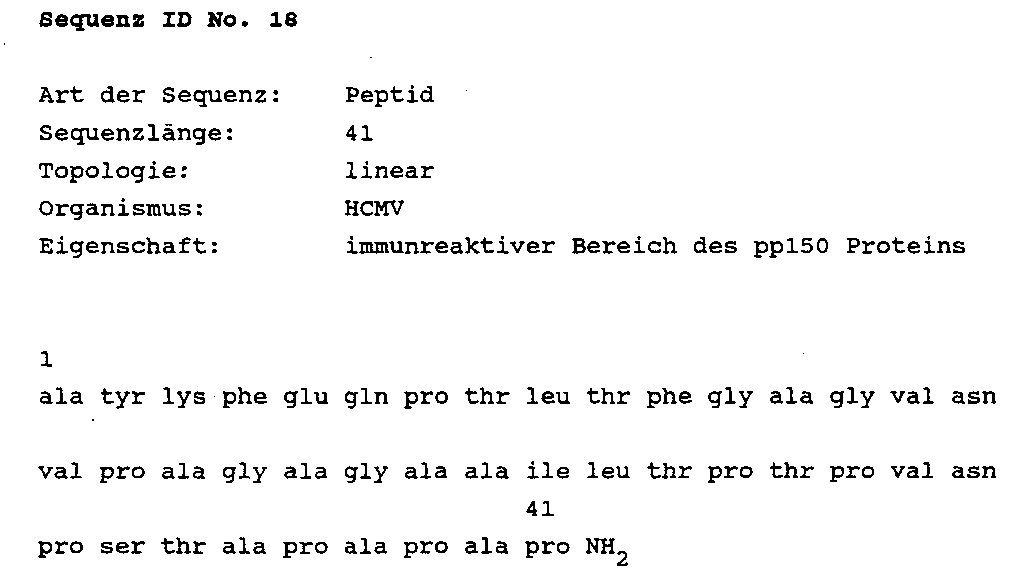

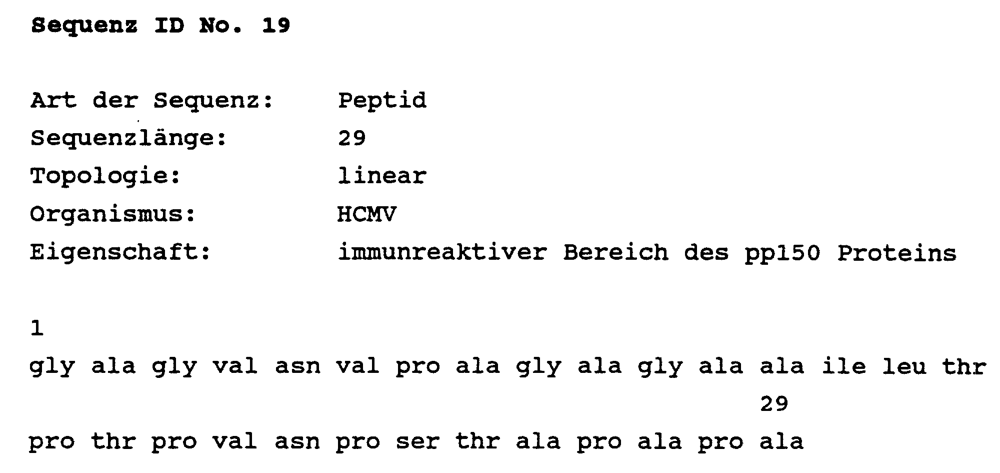

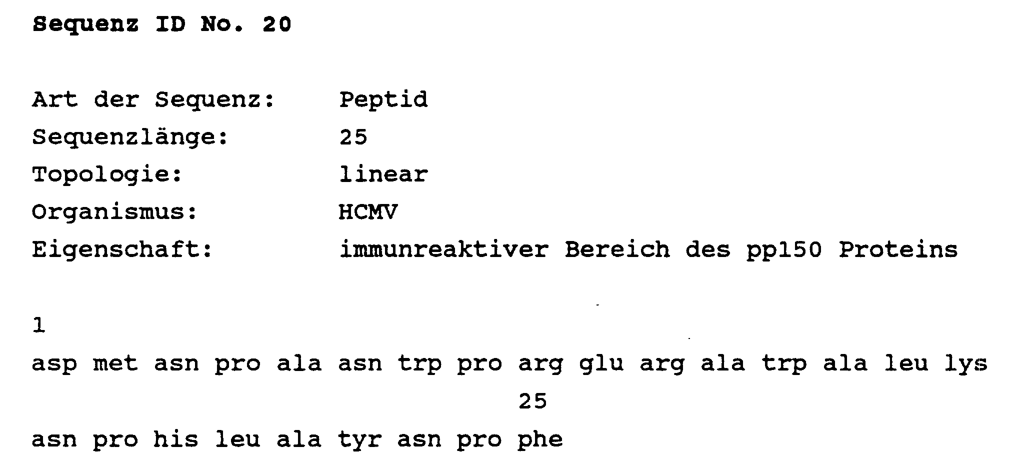

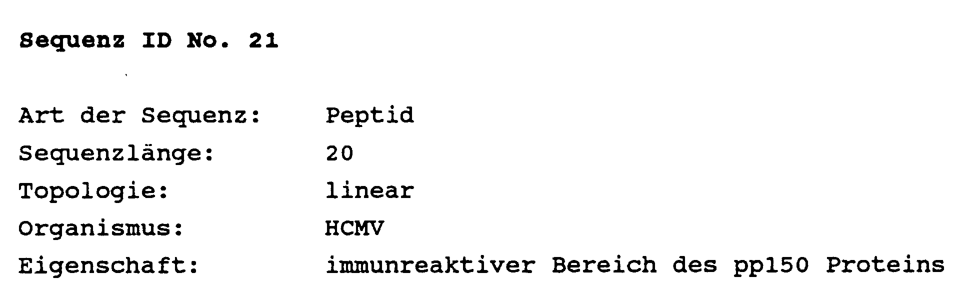

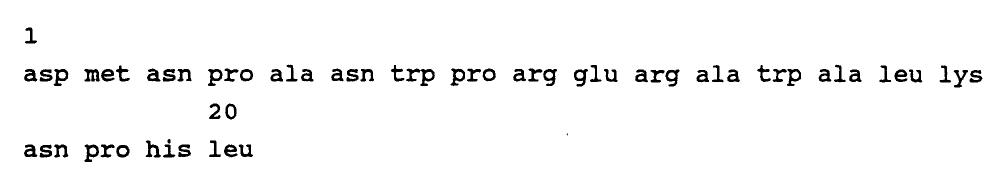

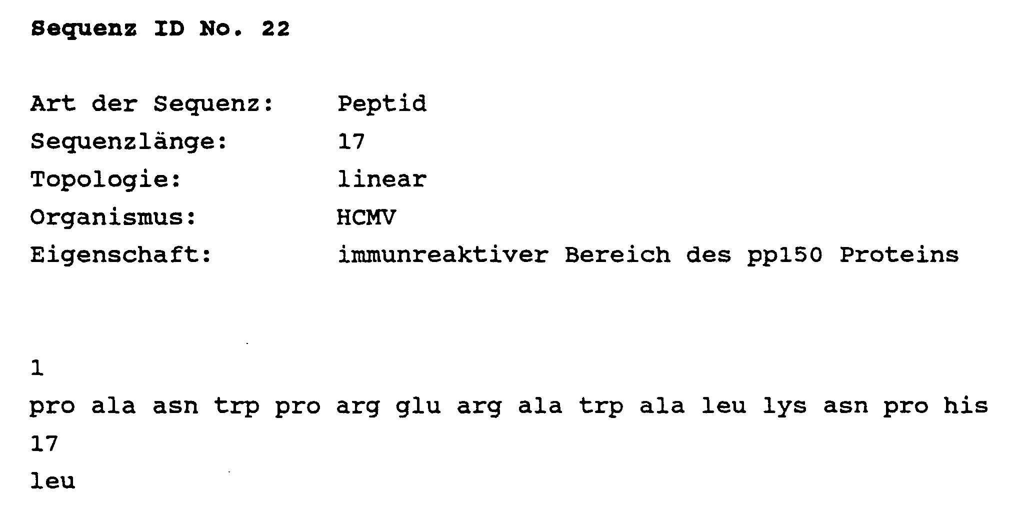

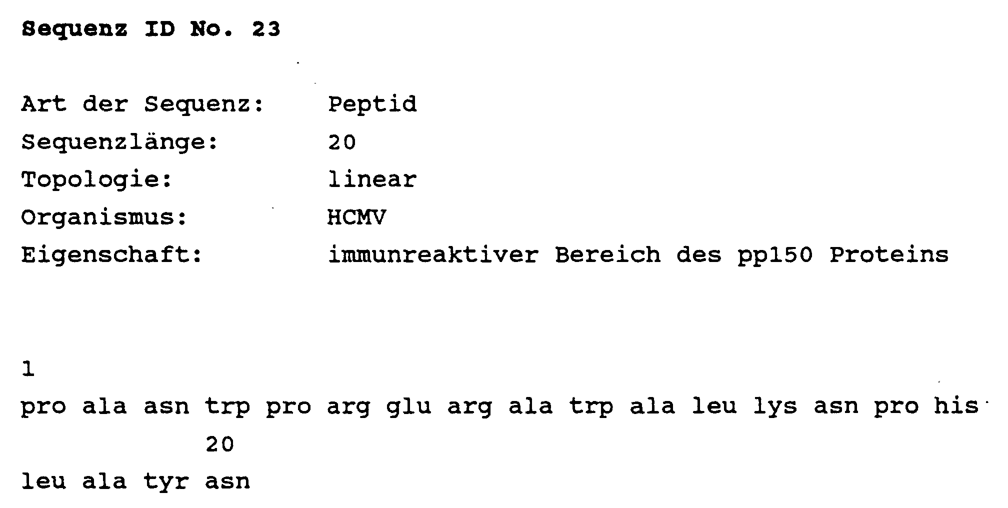

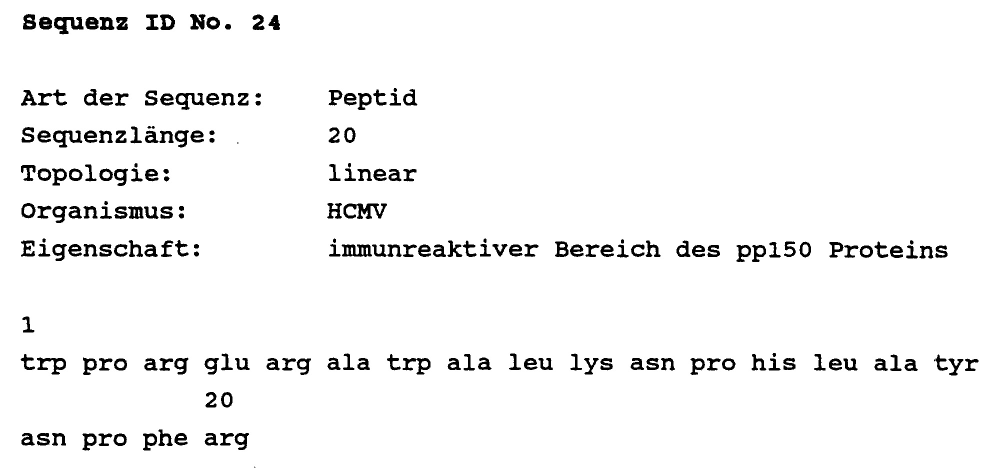

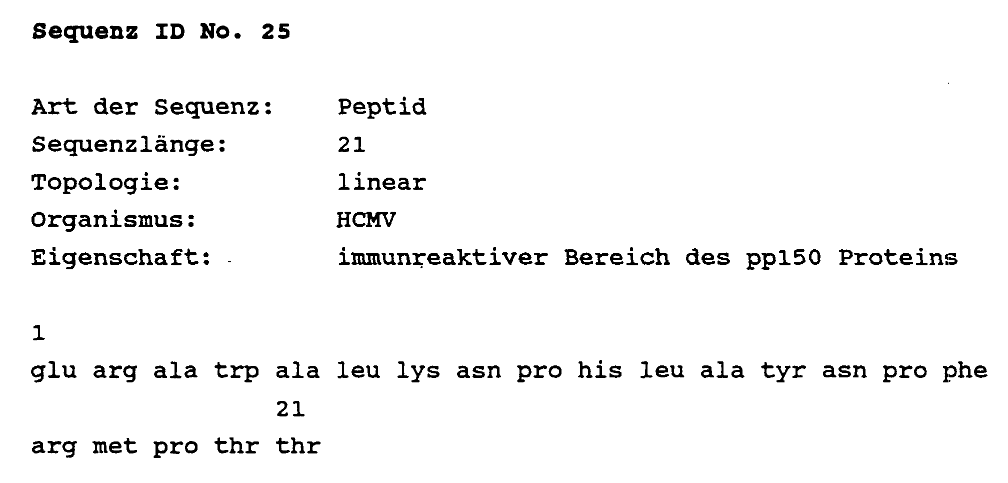

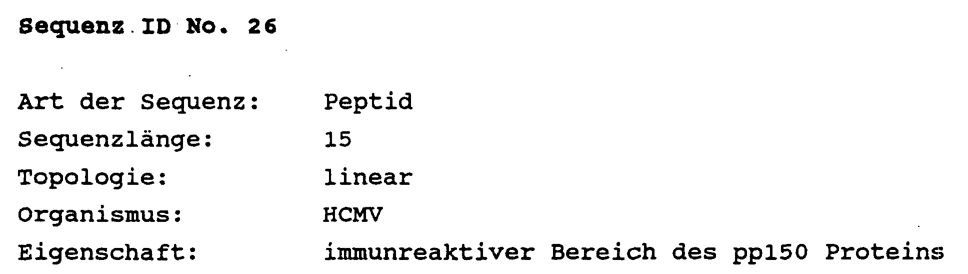

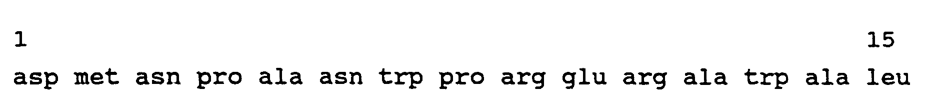

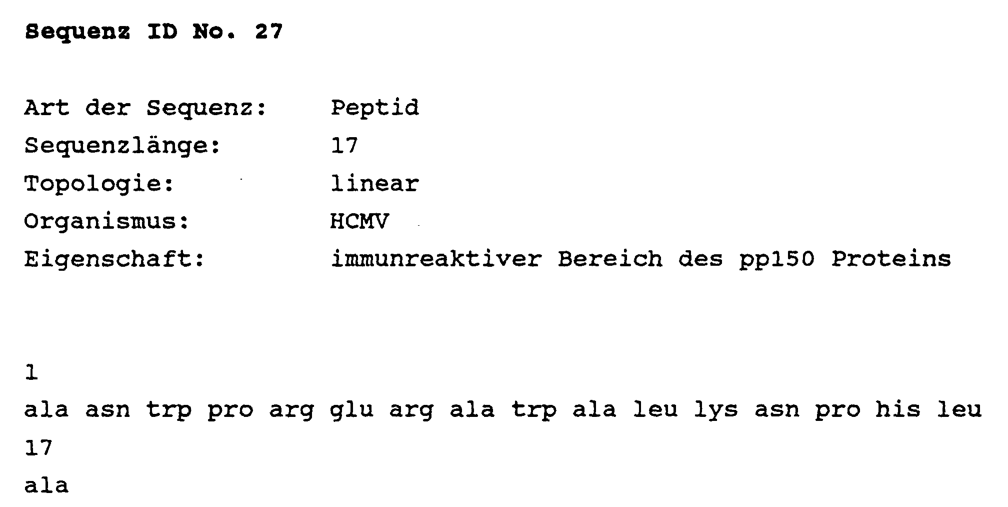

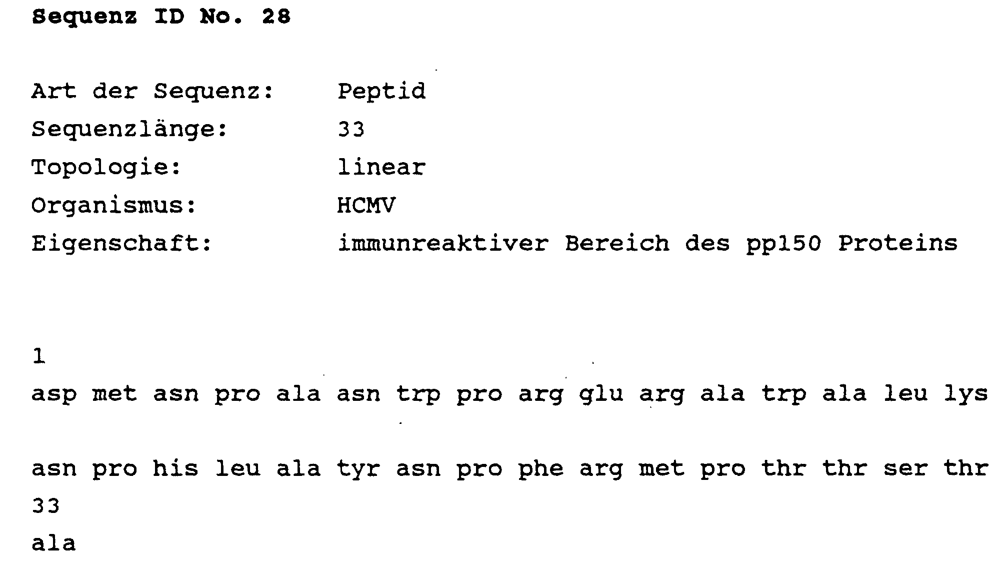

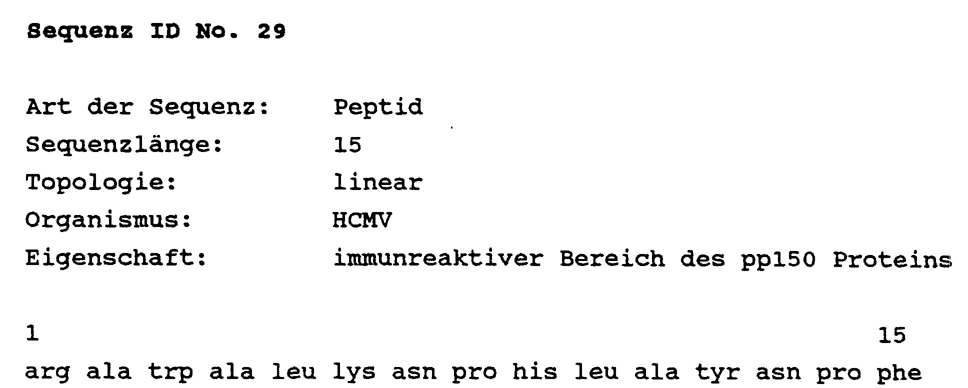

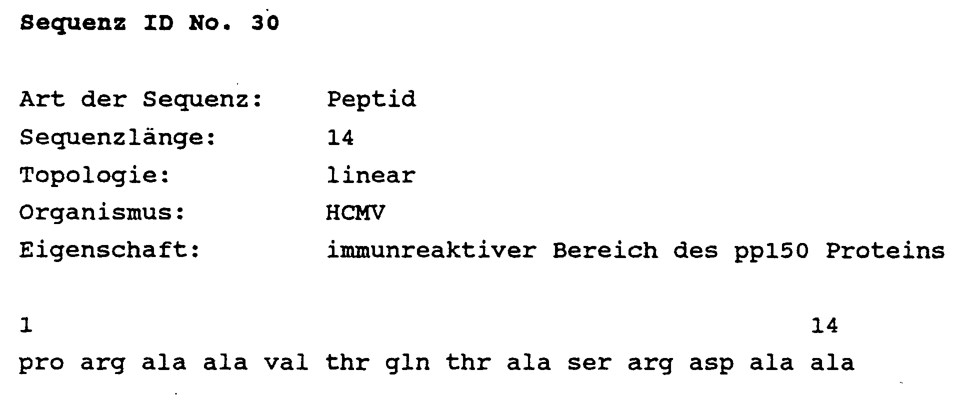

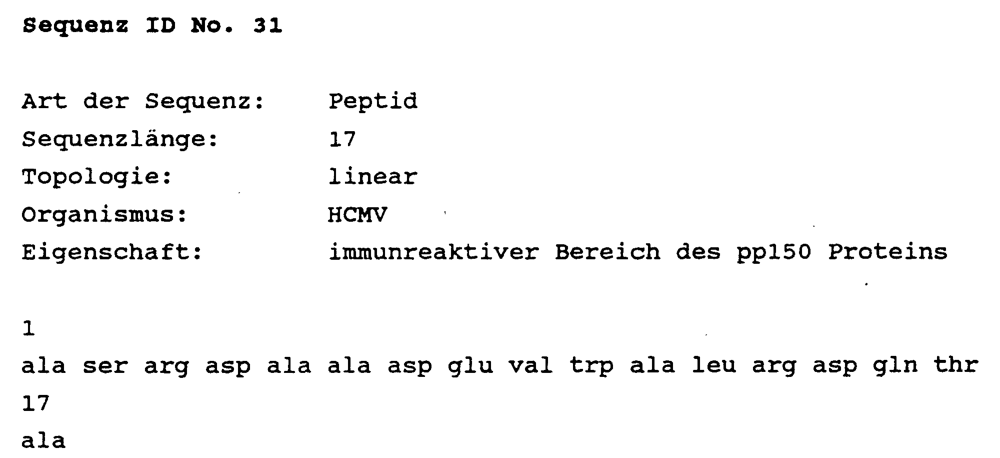

## Patentansprüche

1. Peptide, die spezifisch mit Antikörpern gegen HCMV reagieren, **dadurch gekennzeichnet, daß** sie mindestens eine der folgenden Aminosäuresequenzen enthalten ASe-gly ala gly ala ala ile leu-BSm     (Peptid 1) CSₙ-arg ala trp ala leu-DSₒ (Peptid 2) und/oder ES_p-ala ser arg asp ala ala-FS_q (Peptid 3) wobei AS, BS, CS, DS, ES und FS unabhängig voneinander eine beliebige Aminosäure bedeuten und
e unabhängig voneinander ganze Zahlen von 0 bis 22,
m " " " " " 0 bis 25,
n und o " " " " " 0 bis 18
und
p und q " " " " " 0 bis 11
sind, mit der Maßgabe, daß sie nicht mit den Peptiden Nr. 53 oder 56 gemäß Novak et al. (1991), J. Gen. Virol. 72, S.1409-1413 identisch sind.

2. Peptide nach Anspruch 1, **dadurch gekennzeichnet, daß** sie mindestens eine der folgenden Aminosäuresequenzen enthalten und zwar
Zu Peptid 1: 
1.1
ala tyr lys phe glu gln pro thr leu thr phe gly ala gly val asn val pro ala
gly ala gly ala ala ile leu
1.2
phe glu gln pro thr leu thr phe gly ala gly val asn val pro ala gly ala gly
ala ala ile leu thr pro thr pro val
1.3
phe glu gln pro thr leu thr phe gly ala gly val asn val pro ala gly ala gly
ala ala ile leu thr pro thr pro val asn pro ser thr ala
 
1.4
phe glu gln pro thr thr leu thr phe gly ala gly val asn val pro ala gly ala
gly ala ala ile leu thr pro thr pro val asn pro ser thr ala pro ala pro ala
1.5
pro thr leu thr phe gly ala gly val asn val pro ala gly ala gly ala ala ile
leu thr pro thr pro val asn pro ser thr ala pro ala pro ala
1.6
pro thr leu thr phe gly ala gly val asn val pro ala gly ala gly ala ala ile
leu thr pro thr pro val asn pro ser thr ala pro ala pro ala pro NH2
1.7
val asn val pro ala gly ala gly ala ala ile leu thr pro thr pro val asn pro
1.8
pro ala gly ala gly ala ala ile leu thr pro thr pro val asn pro ser thr ala
pro ala
1.9
val asn val pro ala gly ala gly ala ala ile leu thr pro thr pro val asn pro
ser thr ala pro ala pro ala pro thr pro thr phe
1.10
pro ala gly ala gly ala ala ile leu thr pro thr üpro val asn pro ser thr ala
pro ala pro ala pro thr pro thr phe ala gly thr
1.11
gly ala gly val asn val pro ala gly ala gly ala ala ile leu thr pro thr pro val
asn pro ser thr ala pro ala pro ala pro NH2
1.12
gly ala gly ala ala ile leu thr pro thr pro val asn pro ser thr ala pro ala
pro ala pro thr pro thr phe ala gly thr gln thr pro
1.13
ala tyr lys phe glu gln pro thr leu thr phe gly ala gly val asn val pro ala
gly ala gly ala ala ile leu thr pro thr pro val asn pro ser thr ala pro ala
pro ala pro
1.14
ala tyr lys phe glu gln pro thr leu thr phe gly ala gly val asn val pro ala
gly ala gly ala ala ile leu thr pro thr pro val asn pro ser thr ala pro ala
pro ala pro thr pro thr phe ala gly thr gln thr pro
1.15
ala tyr lys phe glu gln pro thr leu thr phe gly ala gly val asn val pro ala
gly ala gly ala ala ile leu thr pro thr pro val asn pro ser thr ala pro ala
pro ala pro NH2
 
1.16
gly ala gly val asn val pro ala gly ala gly ala ala ile leu thr pro thr pro val
asn pro ser thr ala pro ala pro ala
 Zu Peptid 2: 
2.1
asp met asn pro ala asn trp pro arg glu arg ala trp ala leu lys asn pro
his leu ala tyr asn pro phe
2.2
asp met asn pro ala asn trp pro arg glu arg ala trp ala leu lys asn pro
his leu
2.3
pro ala asn trp pro arg glu arg ala trp ala leu lys asn pro his leu
2.4
pro ala asn trp pro arg glu arg ala trp ala leu lys asn pro his leu ala tyr
asn
2.5
trp pro arg glu arg ala trp ala leu lys asn pro his leu ala tyr asn pro phe
arg
2.6
glu arg ala trp ala leu lys asn pro his leu ala tyr asn pro phe arg met pro
thr thr
2.7
asp met asn pro ala asn trp pro arg glu arg ala trp ala leu
2.8
ala asn trp pro arg glu arg ala trp ala leu lys asn
pro his leu ala
2.9
asp met asn pro ala asn trp pro arg glu arg ala trp ala leu lys asn pro
his leu ala tyr asn pro phe arg met pro thr thr ser thr ala
2.10
arg ala trp ala leu lys asn pro his leu ala tyr asn pro phe
 Zu Peptid 3:

3.1
pro arg ala ala val thr gln thr ala ser arg asp ala ala
3.2
ala ser arg asp ala ala asp glu val trp ala leu arg asp gln thr ala

3. Peptid nach einem der Ansprüche 1 oder 2, **dadurch gekennzeichnet, daß** es in folgender Weise derivatisiert wird:
a) aminoterminale oder carboxyterminale Angliederung von 1 bis 40, vorzugsweise 1 bis 20 oder insbesondere 1 bis 10 Amonosäuren, vorzugsweise Cystein zur Verknüpfung von Peptiden untereinander ode an einen Träger; oder
b) Austausch einer Aminosäure durch eine andere Aminosäure innerhalb folgender Gruppen: Gly, Ala; Val, Ile, Leu; Asp, Glu; Asn, Gln; Ser, Thr; Lys, Arg; Phe, Tyr; Ala, Ser; Ala, Thr; Ala, Val; Ala, Pro; Ala, Glu; Leu, Gln; Gly, Phe; Ile, Ser und Ile, Met; oder
c) Zusatz einer hydrophoben Sequenz, umfassend 2 bis 20 hydrophobe Aminosäuren, beispielsweise Phe-Ala-Phe-Ala-Phe zur Verbesserung der Adsorptionseigenschaften des Peptids an einen Träger.

4. Peptide nach mindestens einem der Ansprüche 1 bis 3, **dadurch gekennzeichnet, daß** sie an einen Träger gebunden sind.

5. Peptidmischungen, die Peptide nach mindestens einem der Ansprüche 1 bis 4 enthalten.

6. Peptidmischungen nach Anspruch 5, **dadurch gekennzeichnet, daß** die Peptide untereinander direkt oder über einen Spacer verknüpft sind.

7. Peptide oder Peptidmischungen nach mindestens einem der Ansprüche 1 bis 6, **dadurch gekennzeichnet, daß** sie peptidchemisch synthetisiert sind.

8. DNA-Fragmente, die für mindestens eines der Peptide nach Anspruch 1 bis 3 codieren.

9. Immunchemisches Verfahren zum Nachweis und/oder zur Bestimmung von HCMV Antikörper unter Verwendung von Peptiden nach mindestens einem der Ansprüche 1 bis 7.

10. Verfahren nach Anspruch 9, **dadurch gekennzeichnet, daß** die Peptide untereinander direkt oder über einen Spacer verknüpft sind.

11. Verfahren nach Anspruch 10, **dadurch gekennzeichnet, daß** der Spacer humanes Serumalbumin und/oder Polylysin ist.

12. Verfahren nach mindestens einem der Ansprüche 9 bis 11, **dadurch gekennzeichnet, daß** die Peptide an einen Träger gebunden sind.

13. Verfahren nach Anspruch 12, **dadurch gekennzeichnet, daß** der Träger ausgewählt wird aus der Gruppe bestehend aus Polystyrol, Polyvinylchlorid, Polyamid und anderen synthetischen Polymeren, natürlichen Polymeren wie Zellulose sowie derivatisierten natürlichen Polymeren wie Zelluloseacetat und Nitrozellulose als auch Glas, insbesondere als Glasfaser.

14. Verfahren nach Anspruch 13, **dadurch gekennzeichnet, daß** der Träger Polystyrol ist.

15. Verfahren nach mindestens einem der Ansprüche 9 bis 14, **dadurch gekennzeichnet, daß** der Nachweis und/oder die Bestimmung des epitop-gebundenen Antikörpers mit Hilfe von enzym-markierten, fluoreszenz-markierten, chemilumineszenz-markierten, biotin-markierten oder radioaktiv-markierten Antikörpern gegen die epitop-gebundenen Antikörper erfolgt.

16. Analytisches Verfahren zum Nachweis und/oder zur Bestimmung von HCMV, **dadurch gekennzeichnet, daß** als spezifischer Schritt eine Hybridisierungsreaktion eingesetzt wird, in der mindestens eine Nukleinsäuresonde verwendet wird, die in ihrem spezifischen Teil komplementär zu mindestens einer der DNA Sequenzen nach Anspruch 8, jedoch nicht zum kompletten im Vektor pXP1 enthaltenen Xholl-Pstl-Fragment ist.

17. Immunologisches Testbesteck zum Nachweis und/oder Bestimmung von HCMV Antikörpern, wobei das Testbesteck einen oder mehrere Träger enthält, an dem ein oder mehrere Epitope der Peptide oder Peptidmischungen nach mindestens einem der Ansprüche 1 bis 7 fixiert sind.

18. Testbesteck nach Anspruch 17, **dadurch gekennzeichnet, daß** der Träger ausgewählt wird aus der Gruppe bestehend aus Polystyrol, Polyvinylchlorid, Polyamid und anderen synthetischen Polymeren, natürlichen Polymeren wie Zellulose sowie derivatisierten natürlichen Polymeren wie Zelluloseacetat und Nitrozellulose als auch Glas, insbesondere als Glasfaser.

19. Testbesteck nach Anspruch 18, **dadurch gekennzeichnet, daß** der Träger Polystyrol ist.

20. Testbesteck nach mindestens einem der Ansprüche 17 - 19, **dadurch gekennzeichnet, daß** der gleichzeitige Nachweis und/oder die gleichzeitige Bestimmung mit Hilfe von enzym-markierten, fluoreszenzmarkierten, chemilumineszenz-markierten, biotin-markierten oder radioaktiv-markierten Antikörpern gegen die epitop-gebundenen Antikörper erfolgt.

21. Testbesteck nach Anspruch 20, **dadurch gekennzeichnet, daß** als enzymmarkierte Antikörper die Enzyme alkalische Phosphatase und/oder Meerrettich Peroxidase verwendet werden.

22. Immunchemisches Kombinationsverfahren zum Nachweis und/oder zur Bestimmung von mehreren verschiedenen Antikörperspezifitäten gegen jeweils unterschiedliche Pathogene, wobei ein oder mehrere Peptide nach mindestens einem der Ansprüche 1 bis 7 und ein oder mehrere immunreaktive Peptide von anderen Pathogenen als HCMV verwendet werden.

23. Immunchemisches Kombinationsverfahren nach Anspruch 22, **dadurch gekennzeichnet, daß** als immunreaktive Peptide von anderen Pathogenen Peptide von HSV 1/2, EBV, VZV, HHV6, HAV, HBV, HCV und/oder HIV 1/2 oder immunreaktive Teile davon verwendet werden.

24. Immunchemisches Kombinationsverfahren nach Anspruch 22 oder 23,
**dadurch gekennzeichnet, daß** das Verfahren differenzierend durchgeführt wird.

25. Kombinationstestbesteck zum Nachweis und/oder zur Bestimmung von mehreren verschiedenen Antikörperspezifitäten gegen jeweils unterschiedliche Pathogene, wobei ein oder mehrere Peptide nach mindestens einem der Ansprüche 1 bis 7 und ein oder mehrere immunreaktive Peptide von anderen Pathogenen als HCMV an einen oder mehrere Träger fixiert sind.

26. Vaccine, die mindestens ein Peptid oder eine Mischung von Peptiden nach mindestens einem der Ansprüche 1 - 7 enthält.

27. Verwendung von Peptiden oder Mischungen von Peptiden nach mindestens einem der Ansprüche 1 - 7 zur Herstellung und Gewinnung von Antikörpern durch Immunisieren von Säugetieren, insbesondere Kaninchen.

28. Verwendung der nach dem Verfahren gemäß Anspruch 27 erzeugten Antikörper in einem diagnostischen Verfahren in vitro.

29. Verwendung gemäß Anspruch 28, **dadurch gekennzeichnet, daß** das diagnostische Verfahren ein heterogener Immuntest ist.

## Patentansprüche (Patentansprüche für folgende(n) Vertragsstaat(en): ES)

1. Verfahren zur gentechnischen oder peptidchemischen Herstellung von Peptiden, die spezifisch mit Antikörpern gegen HCMV reagieren,
**dadurch gekennzeichnet, daß** sie mindestens eine der folgenden Aminosäuresequenzen enthalten ASe-gly ala gly ala ala ile leu-BSm (Peptid 1) CSₙ-arg ala trp ala leu-DSₒ (Peptid 2) und/oder ES_p-ala ser arg asp ala ala-FS_q (Peptid 3) wobei AS, BS, CS, DS, ES und FS unabhängig voneinander eine beliebige Aminosäure bedeuten und
e unabhängig voneinander ganze Zahlen von 0 bis 22,
m " " " " " 0 bis 25,
n und o " " " " " 0 bis 18
und
p und q " " " " " 0 bis 11
sind, mit der Maßgabe, daß sie nicht mit den Peptiden Nr. 53 oder 56 gemäß Novak et al. (1991), J. Gen. Virol. 72, S.1409-1413 identisch sind.

2. Verfahren nach Anspruch 1, **dadurch gekennzeichnet, daß** die Peptide mindestens eine der folgenden Aminosäuresequenzen enthalten und zwar
Zu Peptid 1: 
1.1
ala tyr lys phe glu gln pro thr leu thr phe gly ala gly val asn val pro ala
gly ala gly ala ala ile leu
1.2
phe glu gln pro thr leu thr phe gly ala gly val asn val pro ala gly ala gly
ala ala ile leu thr pro thr pro val
 
1.3
phe glu gln pro thr leu thr phe gly ala gly val asn val pro ala gly ala gly
ala ala ile leu thr pro thr pro val asn pro ser thr ala
1.4
phe glu gln pro thr thr leu thr phe gly ala gly val asn val pro ala gly ala
gly ala ala ile leu thr pro thr pro val asn pro ser thr ala pro ala pro ala
1.5
pro thr leu thr phe gly ala gly val asn val pro ala gly ala gly ala ala ile
leu thr pro thr pro val asn pro ser thr ala pro ala pro ala
1.6
pro thr leu thr phe gly ala gly val asn val pro ala gly ala gly ala ala ile
leu thr pro thr pro val asn pro ser thr ala pro ala pro ala pro NH2
1.7
val asn val pro ala gly ala gly ala ala ile leu thr pro thr pro val asn pro
1.8
pro ala gly ala gly ala ala ile leu thr pro thr pro val asn pro ser thr ala
pro ala
1.9
val asn val pro ala gly ala gly ala ala ile leu thr pro thr pro val asn pro
ser thr ala pro ala pro ala pro thr pro thr phe
1.10
pro ala gly ala gly ala ala ile leu thr pro thr pro val asn pro ser thr ala
pro ala pro ala pro thr pro thr phe ala gly thr
1.11
gly ala gly val asn val pro ala gly ala gly ala ala ile leu thr pro thr pro val
asn pro ser thr ala pro ala pro ala pro NH2
1.12
gly ala gly ala ala ile leu thr pro thr pro val asn pro ser thr ala pro ala
pro ala pro thr pro thr phe ala gly thr gln thr pro
1.13
ala tyr lys phe glu gln pro thr leu thr phe gly ala gly val asn val pro ala
gly ala gly ala ala ile leu thr pro thr pro val asn pro ser thr ala pro ala
pro ala pro
1.14
ala tyr lys phe glu gln pro thr leu thr phe gly ala gly val asn val pro ala
gly ala gly ala ala ile leu thr pro thr pro val asn pro ser thr ala pro ala
pro ala pro thr pro thr phe ala gly thr gln thr pro
 
1.15
ala tyr lys phe glu gln pro thr leu thr phe gly ala gly val asn val pro ala
gly ala gly ala ala ile leu thr pro thr pro val asn pro ser thr ala pro ala
pro ala pro NH2
1.16
gly ala gly val asn val pro ala gly ala gly ala ala ile leu thr pro thr pro val
asn pro ser thr ala pro ala pro ala
 Zu Peptid 2: 
2.1
asp met asn pro ala asn trp pro arg glu arg ala trp ala leu lys asn pro
his leu ala tyr asn pro phe
2.2
asp met asn pro ala asn trp pro arg glu arg ala trp ala leu lys asn pro
his leu
2.3
pro ala asn trp pro arg glu arg ala trp ala leu lys asn pro his leu
2.4
pro ala asn trp pro arg glu arg ala trp ala leu lys asn pro his leu ala tyr
asn
2.5
trp pro arg glu arg ala trp ala leu lys asn pro his leu ala tyr asn pro phe
arg
2.6
glu arg ala trp ala leu lys asn pro his leu ala tyr asn pro phe arg met pro
thr thr
2.7
asp met asn pro ala asn trp pro arg glu arg ala trp ala leu
2.8
ala asn trp pro arg glu arg ala trp ala leu lys asn
pro his leu ala
2.9
asp met asn pro ala asn trp pro arg glu arg ala trp ala leu lys asn pro
his leu ala tyr asn pro phe arg met pro thr thr ser thr ala
2.10
arg ala trp ala leu lys asn pro his leu ala tyr asn pro phe
 Zu Peptid 3:

3.1
pro arg ala ala val thr gln thr ala ser arg asp ala ala
3.2
ala ser arg asp ala ala asp glu val trp ala leu arg asp gln thr ala

3. Verfahren nach einem der Ansprüche 1 oder 2, **dadurch gekennzeichnet, daß** das Peptid in folgender Weise derivatisiert wird:
a) aminoterminale oder carboxyterminale Angliederung von 1 bis 40, vorzugsweise 1 bis 20 oder insbesondere 1 bis 10 Aminosäuren, vorzugsweise Cystein zur Verknüpfung von Peptiden untereinander oder an einen Träger; oder
b) Austausch einer Aminosäure durch eine andere Aminosäure innerhalb folgender Gruppen: Gly, Ala; Val, Ile, Leu; Asp, Glu; Asn, Gln; Ser, Thr; Lys, Arg; Phe, Tyr; Ala, Ser; Ala, Thr; Ala, Val; Ala, Pro; Ala, Glu; Leu, Gln; Gly, Phe; Ile, Ser und Ile, Met; oder
c) Zusatz einer hydrophoben Sequenz, umfassend 2 bis 20 hydrophobe Aminosäuren, beispielsweise Phe-Ala-Phe-Ala-Phe zur Verbesserung der Adsorptionseigenschaften des Peptids an einen Träger.

4. Verfahren nach mindestens einem der Ansprüche 1 bis 3, **dadurch gekennzeichnet, daß** die Peptide an einen Träger gebunden sind.

5. Verfahren zur Herstellung von Peptidmischungen, **dadurch gekennzeichnet, daß** Peptide hergestellt nach einem Verfahren nach mindestens einem der Ansprüche 1 bis 4 verwendet werden.

6. Verfahren nach Anspruch 5, **dadurch gekennzeichnet, daß** die Peptide untereinander direkt oder über einen Spacer verknüpft sind.

7. Verfahren nach mindestens einem der Ansprüche 1 bis 6, **dadurch gekennzeichnet, daß** die Peptide peptidchemisch synthetisiert sind.

8. Verfahren zur Herstellung von DNA-Fragmenten, die für mindestens eines der Peptide nach Anspruch 1 bis 3 codieren, **dadurch gekennzeichnet, daß** die DNA-Fragmente gentechnisch oder chemisch synthetisiert werden.

9. Immunchemisches Verfahren zum Nachweis und/oder zur Bestimmung von HCMV-Antikörpern unter Verwendung von Peptiden nach mindestens einem der Ansprüche 1 bis 7.

10. Verfahren nach Anspruch 9, **dadurch gekennzeichnet, daß** die Peptide untereinander direkt oder über einen Spacer verknüpft sind.

11. Verfahren nach Anspruch 10, **dadurch gekennzeichnet, daß** der Spacer humanes Serumalbumin und/oder Polylysin ist.

12. Verfahren nach mindestens einem der Ansprüche 9 bis 11, **dadurch gekennzeichnet, daß** die Peptide an einen Träger gebunden sind.

13. Verfahren nach Anspruch 12, **dadurch gekennzeichnet, daß** der Träger ausgewählt wird aus der Gruppe bestehend aus Polystyrol, Polyvinylchlorid, Polyamid und anderen synthetischen Polymeren, natürlichen Polymeren wie Zellulose sowie derivatisierten natürlichen Polymeren wie Zelluloseacetat und Nitrozellulose, als auch Glas, insbesondere als Glasfaser.

14. Verfahren nach Anspruch 13, **dadurch gekennzeichnet, daß** der Träger Polystyrol ist.

15. Verfahren nach mindestens einem der Ansprüche 9 bis 14, **dadurch gekennzeichnet, daß** der Nachweis und/oder die Bestimmung des epitopgebundenen Antikörpers mit Hilfe von enzymmarkierten, fluoreszenzmarkierten, chemilumineszenzmarkierten, biotinmarkierten oder radioaktiv markierten Antikörpern gegen die epitopgebundenen Antikörper erfolgt.

16. Analytisches Verfahren zum Nachweis und/oder zur Bestimmung von HCMV, **dadurch gekennzeichnet, daß** als spezifischer Schritt eine Hybridisierungsreaktion eingesetzt wird, in der mindestens eine Nukleinsäuresonde verwendet wird, die in ihrem spezifischen Teil komplementär zu mindestens einer der DNA Sequenzen nach Anspruch 8, jedoch nicht zum kompletten im Vektor pXP1 enthaltenen Xholl-Pstl-Fragment ist.

17. Verfahren zur Herstellung eines immunologischen Testbestecks zum Nachweis und/oder Bestimmung von HCMV Antikörpern, wobei das Testbesteck einen oder mehrere Träger enthält, an dem ein oder mehrere Epitope der Peptide oder Peptidmischungen nach mindestens einem der Ansprüche 1 bis 7 fixiert sind.

18. Verfahren nach Anspruch 17, **dadurch gekennzeichnet, daß** der Träger ausgewählt wird aus der Gruppe bestehend aus Polystyrol, Polyvinylchlorid, Polyamid und anderen synthetischen Polymeren, natürlichen Polymeren wie Zellulose sowie derivatisierten natürlichen Polymeren wie Zelluloseacetat und Nitrozellulose als auch Glas, insbesondere als Glasfaser.

19. Verfahren nach Anspruch 18, **dadurch gekennzeichnet, daß** der Träger Polystyrol ist.

20. Verfahren nach mindestens einem der Ansprüche 17 - 19, **dadurch gekennzeichnet, daß** der gleichzeitige Nachweis und/oder die gleichzeitige Bestimmung mit Hilfe von enzymmarkierten, fluoreszenzmarkierten, chemilumineszenzmarkierten, biotinmarkierten oder radioaktiv markierten Antikörpern gegen die epitopgebundenen Antikörper erfolgt.

21. Verfahren nach Anspruch 20, **dadurch gekennzeichnet, daß** als enzymmarkierte Antikörper die Enzyme alkalische Phosphatase und/oder Meerrettich-Peroxidase verwendet werden.

22. Immunchemisches Kombinationsverfahren zum Nachweis und/oder zur Bestimmung von mehreren verschiedenen Antikörperspezifitäten gegen jeweils unterschiedliche Pathogene, wobei ein oder mehrere Peptide nach mindestens einem der Ansprüche 1 bis 7 und ein oder mehrere immunreaktive Peptide von anderen Pathogenen als HCMV verwendet werden.

23. Immunchemisches Kombinationsverfahren nach Anspruch 22, **dadurch gekennzeichnet, daß** als immunreaktive Peptide von anderen Pathogenen Peptide von HSV 1/2, EBV, VZV, HHV6, HAV, HBV, HCV und/oder HIV 1/2 oder immunreaktive Teile davon verwendet werden.

24. Immunchemisches Kombinationsverfahren nach Anspruch 22 oder 23, **dadurch gekennzeichnet, daß** das Verfahren differenzierend durchgeführt wird.

25. Verfahren zur Herstellung eines Kombinationstestbestecks zum Nachweis und/oder zur Bestimmung von mehreren verschiedenen Antikörperspezifitäten gegen jeweils unterschiedliche Pathogene, wobei ein oder mehrere Peptide nach mindestens einem der Ansprüche 1 bis 7 und ein oder mehrere immunreaktive Peptide von anderen Pathogenen als HCMV an einen oder mehrere Träger fixiert werden.

26. Verfahren zur Herstellung einer Vaccine, **dadurch gekennzeichnet, daß** mindestens ein Peptid oder eine Mischung von Peptiden, hergestellt nach mindestens einem der Ansprüche 1 - 7, verwendet werden.

27. Verwendung von Peptiden oder Mischungen von Peptiden nach mindestens einem der Ansprüche 1 - 7 zur Herstellung und Gewinnung von Antikörpern durch Immunisieren von Säugetieren, insbesondere Kaninchen.

28. Verwendung der nach dem Verfahren gemäß Anspruch 27 erzeugten Antikörper in einem diagnostischen Verfahren in vitro.

29. Verwendung gemäß Anspruch 28, **dadurch gekennzeichnet, daß** das diagnostische Verfahren ein heterogener Immuntest ist.

## Claims

1. A peptide which reacts specifically with antibodies against HCMV, which contains at least one of the following amino-acid sequences ASe-gly ala gly ala ala ile leu-BSm     (peptide 1) CSn-arg ala trp ala leu-DSo (peptide 2) and/or ESp-ala ser arg asp ala ala-FSq (peptide 3) where AS, BS, CS, DS, ES and FS are, independently of one another, any appropriate amino acid and
e are, independently of one another, integers from 0 to 22,
m " " " " from 0 to 25,
n and o " " " " from 0 to 18
and p and q " " " "
from 0 to 11,
with the proviso that it is not identical to peptides No. 53 or 56 according to Novak et al. (1991), J. Gen. Virol. 72, pp. 1409-1413.

2. A peptide as claimed in claim 1, which contains at least one of the following amino-acid sequences
for peptide 1: 
1.1
ala tyr lys phe glu gln pro thr leu thr phe gly ala gly val asn val pro ala
gly ala gly ala ala ile leu
1.2
phe glu gln pro thr leu thr phe gly ala gly val asn val pro ala gly ala gly
ala ala ile leu thr pro thr pro val
1.3
phe glu gln pro thr leu thr phe gly ala gly val asn val pro ala gly ala gly
ala ala ile leu thr pro thr pro val asn pro ser thr ala
 
1.4
phe glu gln pro thr thr leu thr phe gly ala gly val asn val pro ala gly ala
gly ala ala ile leu thr pro thr pro val asn pro ser thr ala pro ala pro ala
1.5
pro thr leu thr phe gly ala gly val asn val pro ala gly ala gly ala ala ile
leu thr pro thr pro val asn pro ser thr ala pro ala pro ala
1.6
pro thr leu thr phe gly ala gly val asn val pro ala gly ala gly ala ala ile
leu thr pro thr pro val asn pro ser thr ala pro ala pro ala pro NH2
1.7
val asn val pro ala gly ala gly ala ala ile leu thr pro thr pro val asn pro
1.8
pro ala gly ala gly ala ala ile leu thr pro thr pro val asn pro ser thr ala
pro ala
1.9
val asn val pro ala gly ala gly ala ala ile leu thr pro thr pro val asn pro
ser thr ala pro ala pro ala pro thr pro thr phe
1.10
pro ala gly ala gly ala ala ile leu thr pro thr üpro val asn pro ser thr ala
pro ala pro ala pro thr pro thr phe ala gly thr
1.11
gly ala gly val asn val pro ala gly ala gly ala ala ile leu thr pro thr pro val
asn pro ser thr ala pro ala pro ala pro NH2
1.12
gly ala gly ala ala ile leu thr pro thr pro val asn pro ser thr ala pro ala
pro ala pro thr pro thr phe ala gly thr gln thr pro
1.13
ala tyr lys phe glu gln pro thr leu thr phe gly ala gly val asn val pro ala
gly ala gly ala ala ile leu thr pro thr pro val asn pro ser thr ala pro ala
pro ala pro
1.14
ala tyr lys phe glu gln pro thr leu thr phe gly ala gly val asn val pro ala
gly ala gly ala ala ile leu thr pro thr pro val asn pro ser thr ala pro ala
pro ala pro thr pro thr phe ala gly thr gln thr pro
1.15
ala tyr lys phe glu gln pro thr leu thr phe gly ala gly val asn val pro ala
gly ala gly ala ala ile leu thr pro thr pro val asn pro ser thr ala pro ala
pro ala pro NH2

1.16
gly ala gly val asn val pro ala gly ala gly ala ala ile leu thr pro thr pro val
asn pro ser thr ala pro ala pro ala
 for peptide 2: 
2.1
asp met asn pro ala asn trp pro arg glu arg ala trp ala leu lys asn pro
his leu ala tyr asn pro phe
2.2
asp met asn pro ala asn trp pro arg glu arg ala trp ala leu lys asn pro
his leu
2.3
pro ala asn trp pro arg glu arg ala trp ala leu lys asn pro his leu
2.4
pro ala asn trp pro arg glu arg ala trp ala leu lys asn pro his leu ala tyr
asn
2.5
trp pro arg glu arg ala trp ala leu lys asn pro his leu ala tyr asn pro phe
arg
2.6
glu arg ala trp ala leu lys asn pro his leu ala tyr asn pro phe arg met pro
thr thr
2.7
asp met asn pro ala asn trp pro arg glu arg ala trp ala leu
2.8
ala asn trp pro arg glu arg ala trp ala leu lys asn
pro his leu ala
2.9
asp met asn pro ala asn trp pro arg glu arg ala trp ala leu lys asn pro
his leu ala tyr asn pro phe arg met pro thr thr ser thr ala
2.10
arg ala trp ala leu lys asn pro his leu ala tyr asn pro phe
 for peptide 3:

3.1
pro arg ala ala val thr gln thr ala ser arg asp ala ala
3.2
ala ser arg asp ala ala asp glu val trp ala leu arg asp gln thr ala

3. A peptide as claimed in either of claims 1 or 2, which is derivatized in the following way:
a) amino-terminal or carboxy-terminal attachment of 1 to 40, preferably 1 to 20 or, in particular, 1 to 10 amino acids, preferably cysteine for linking peptides with one another or to a support; or
b) replacement of one amino acid by another amino acid within the following groups: Gly, Ala; Val, Ile, Leu; Asp, Glu; Asn, Gln; Ser, Thr; Lys, Arg; Phe, Tyr; Ala, Ser; Ala, Thr; Ala, Val; Ala, Pro; Ala, Glu; Leu, Gln; Gly, Phe; Ile, Ser and Ile, Met; or
c) addition of a hydrophobic sequence comprising 2 to 20 hydrophobic amino acids, for example Phe-Ala-Phe-Ala-Phe, to improve the adsorption properties of the peptide to a support.

4. A peptide as claimed in at least one of claims 1 to 3, which is bound to a support.

5. A peptide mixture which contains peptides as claimed in at least one of claims 1 to 4.

6. A peptide mixture as claimed in claim 5, wherein the peptides are linked together directly or via a spacer.

7. A peptide or peptide mixture as claimed in at least one of claims 1 to 6, wherein the peptides have been synthesized chemically.

8. A DNA fragment which codes for at least one of the peptides as claimed in claims 1 to 3.

9. An immunochemical method for the detection and/or for the determination of HCMV antibody using peptides as claimed in at least one of claims 1 to 7.

10. The method as claimed in claim 9, wherein the peptides are linked together directly or via a spacer.

11. The method as claimed in claim 10, wherein the spacer is human serum albumin and/or polylysine.

12. The method as claimed in at least one of claims 9 to 11, wherein the peptides are bound to a support.

13. The method as claimed in claim 12, wherein the support is selected from the group comprising polystyrene, polyvinyl chloride, polyamide and other synthetic polymers, natural polymers such as cellulose, and derivatized natural polymers such as cellulose acetate and nitrocellulose, and glass, especially as glass fibers.

14. The method as claimed in claim 13, wherein the support is polystyrene.

15. The method as claimed in at least one of claims 9 to 14, wherein the detection and/or the determination of the epitope-bound antibody takes place with the aid of enzyme-labeled, fluorescencelabeled, chemiluminescence-labeled, biotin-labeled or radiolabeled antibodies against the epitope-bound antibodies.

16. An analytical method for the detection and/or for the determination of HCMV, which comprises employing as specific step a hybridization reaction which uses at least one nucleic acid probe which in its specific part is complementary to at least one of the DNA sequences as claimed in claim 8 but not to the complete XhoII-PstI fragment contained in the vector pXP1.

17. An immunological assay kit for the detection and/or determination of HCMV antibodies, wherein the assay kit contains one or more supports on which one or more epitopes of the peptides or peptide mixtures as claimed in at least one of claims 1 to 7 are immobilized.

18. The assay kit as claimed in claim 17, wherein the support is selected from the group comprising polystyrene, polyvinyl chloride, polyamide and other synthetic polymers, natural polymers such as cellulose, and derivatized natural polymers such as cellulose acetate and nitrocellulose, and glass, especially as glass fibers.

19. The assay kit as claimed in claim 18, wherein the support is polystyrene.

20. The assay kit as claimed in at least one of claims 17-19, wherein the simultaneous detection and/or the simultaneous determination is carried out with the aid of enzyme-labeled, fluorescence-labeled, chemiluminescence-labeled, biotin-labeled or radiolabeled antibodies against the epitope-bound antibodies.

21. The assay kit as claimed in claim 20, wherein the enzymes alkaline phosphatase and/or horseradish peroxidase are used as enzyme-labeled antibodies.

22. An immunochemical combination method for the detection and/or for the determination of a plurality of different antibody specificities each against different pathogens, wherein one or more peptides as claimed in at least one of claims 1 to 7 and one or more immunoreactive peptides from pathogens other than HCMV are used.

23. The immunochemical combination method as claimed in claim 22, wherein peptides from HSV 1/2, EBV, VZV, HHV6, HAV, HBV, HCV and/or HIV 1/2 or immunoreactive parts thereof are used as immunoreactive peptides from other pathogens.

24. The immunochemical combination method as claimed in claim 22 or 23, which method is carried out differentially.

25. A combination assay kit for the detection and/or for the determination of a plurality of different antibody specificities each against different pathogens, wherein one or more peptides as claimed in at least one of claims 1 to 7 and one or more immunoreactive peptides from pathogens other than HCMV are immobilized on one or more supports.

26. A vaccine which contains at least one peptide or a mixture of peptides as claimed in at least one of claims 1-7.

27. The use of peptides or mixtures of peptides as claimed in at least one of claims 1-7 for producing and obtaining antibodies by immunizing mammals, especially rabbits.

28. The use of the antibodies raised by the method as claimed in claim 27 in a diagnostic method in vitro.

29. The use as claimed in claim 28, wherein the diagnostic method is a heterogeneous immunoassay.

## Claims (Claims for the following Contracting State(s): ES)

1. A process for preparing peptides by genetic engineering or chemically, which peptides react specifically with antibodies against HCMV, wherein said peptides contain at least one of the following. amino-acid sequences ASₑ-gly ala gly ala ala ile leu-BSₘ     (peptide 1) CSₙ-arg ala trp ala leu-DSₒ                    (peptide 2) and/or ESp-ala ser arg asp ala ala-FSq          (peptide 3) where AS, BS, CS, DS, ES and FS are, independently of one another, any appropriate amino acid and
e are, independently of one another, integers from 0 to 22,
m " " " " from 0 to 25,
n and o " " " " from 0 to 18
and p and q " " " " from 0 to 11,
with the proviso that they are not identical to peptides No. 53 or 56 according to Novak et al. (1991), J. Gen. Virol. 72, pp. 1409-1413.

2. A process as claimed in claim 1, wherein the peptides contain at least one of the following amino-acid sequences
for peptide 1: 
1.1
ala tyr lys phe glu gln pro thr leu thr phe gly ala gly val asn val pro ala
gly ala gly ala ala ile leu
1.2
phe glu gln pro thr leu thr phe gly ala gly val asn val pro ala gly ala gly
ala ala ile leu thr pro thr pro val
 
1.3
phe glu gln pro thr leu thr phe gly ala gly val asn val pro ala gly ala gly
ala ala ile leu thr pro thr pro val asn pro ser thr ala
1.4
phe glu gln pro thr thr leu thr phe gly ala gly val asn val pro ala gly ala
gly ala ala ile leu thr pro thr pro val asn pro ser thr ala pro ala pro ala
1.5
pro thr leu thr phe gly ala gly val asn val pro ala gly ala gly ala ala ile
leu thr pro thr pro val asn pro ser thr ala pro ala pro ala
1.6
pro thr leu thr phe gly ala gly val asn val pro ala gly ala gly ala ala ile
leu thr pro thr pro val asn pro ser thr ala pro ala pro ala pro NH2
1.7
val asn val pro ala gly ala gly ala ala ile leu thr pro thr pro val asn pro
1.8
pro ala gly ala gly ala ala ile leu thr pro thr pro val asn pro ser thr ala
pro ala
1.9
val asn val pro ala gly ala gly ala ala ile leu thr pro thr pro val asn pro
ser thr ala pro ala pro ala pro thr pro thr phe
1.10
pro ala gly ala gly ala ala ile leu thr pro thr pro val asn pro ser thr ala
pro ala pro ala pro thr pro thr phe ala gly thr
1.11
gly ala gly val asn val pro ala gly ala gly ala ala ile leu thr pro thr pro val
asn pro ser thr ala pro ala pro ala pro NH2
1.12
gly ala gly ala ala ile leu thr pro thr pro val asn pro ser thr ala pro ala
pro ala pro thr pro thr phe ala gly thr gln thr pro
1.13
ala tyr lys phe glu gln pro thr leu thr phe gly ala gly val asn val pro ala
gly ala gly ala ala ile leu thr pro thr pro val asn pro ser thr ala pro ala
pro ala pro
1.14
ala tyr lys phe glu gln pro thr leu thr phe gly ala gly val asn val pro ala
gly ala gly ala ala ile leu thr pro thr pro val asn pro ser thr ala pro ala
pro ala pro thr pro thr phe ala gly thr gln thr pro
 
1.15
ala tyr lys phe glu gln pro thr leu thr phe gly ala gly val asn val pro ala
gly ala gly ala ala ile leu thr pro thr pro val asn pro ser thr ala pro ala
pro ala pro NH2
1.16
gly ala gly val asn val pro ala gly ala gly ala ala ile leu thr pro thr pro val
asn pro ser thr ala pro ala pro ala
 for peptide 2: 
2.1
asp met asn pro ala asn trp pro arg glu arg ala trp ala leu lys asn pro
his leu ala tyr asn pro phe
2.2
asp met asn pro ala asn trp pro arg glu arg ala trp ala leu lys asn pro
his leu
2.3
pro ala asn trp pro arg glu arg ala trp ala leu lys asn pro his leu
2.4
pro ala asn trp pro arg glu arg ala trp ala leu lys asn pro his leu ala tyr
asn
2.5
trp pro arg glu arg ala trp ala leu lys asn pro his leu ala tyr asn pro phe
arg
2.6
glu arg ala trp ala leu lys asn pro his leu ala tyr asn pro phe arg met pro
thr thr
2.7
asp met asn pro ala asn trp pro arg glu arg ala trp ala leu
2.8
ala asn trp pro arg glu arg ala trp ala leu lys asn
pro his leu ala
2.9
asp met asn pro ala asn trp pro arg glu arg ala trp ala leu lys asn pro
his leu ala tyr asn pro phe arg met pro thr thr ser thr ala
2.10
arg ala trp ala leu lys asn pro his leu ala tyr asn pro phe
 for peptide 3:

3.1
pro arg ala ala val thr gln thr ala ser arg asp ala ala
3.2
ala ser arg asp ala ala asp glu val trp ala leu arg asp gln thr ala

3. A process as claimed in either of claims 1 or 2, wherein the peptide is derivatized in the following way:
a) amino-terminal or carboxy-terminal attachment of 1 to 40, preferably 1 to 20 or, in particular, 1 to 10 amino acids, preferably cysteine for linking peptides with one another or to a support; or
b) replacement of one amino acid by another amino acid within the following groups: Gly, Ala; Val, Ile, Leu; Asp, Glu; Asn, Gln; Ser, Thr; Lys, Arg; Phe, Tyr; Ala, Ser; Ala, Thr; Ala, Val; Ala, Pro; Ala, Glu; Leu, Gln; Gly, Phe; Ile, Ser and Ile, Met; or
c) addition of a hydrophobic sequence comprising 2 to 20 hydrophobic amino acids, for example Phe-Ala-Phe-Ala-Phe, to improve the adsorption properties of the peptide to a support.

4. A process as claimed in at least one of claims 1 to 3, wherein the peptides are bound to a support.

5. A process for preparing peptide mixtures, wherein peptides prepared by a process as claimed in at least one of claims 1 to 4 are used.

6. A process as claimed in claim 5, wherein the peptides are linked together directly or via a spacer.

7. A process as claimed in at least one of claims 1 to 6, wherein the peptides have been synthesized chemically.

8. A process for preparing DNA fragments which code for at least one of the peptides as claimed in claims 1 to 3, wherein the DNA fragments are synthetized by genetic engineering or chemically.

9. An immunochemical method for the detection and/or for the determination of HCMV antibodies using peptides as claimed in at least one of claims 1 to 7.

10. The method as claimed in claim 9, wherein the peptides are linked together directly or via a spacer.

11. The method as claimed in claim 10, wherein the spacer is human serum albumin and/or polylysine.

12. The method as claimed in at least one of claims 9 to 11, wherein the peptides are bound to a support.

13. The method as claimed in claim 12, wherein the support is selected from the group comprising polystyrene, polyvinyl chloride, polyamide and other synthetic polymers, natural polymers such as cellulose, and derivatized natural polymers such as cellulose acetate and nitrocellulose, and glass, especially as glass fibers.

14. The method as claimed in claim 13, wherein the support is polystyrene.

15. The method as claimed in at least one of claims 9 to 14, wherein the detection and/or the determination of the epitope-bound antibody takes place with the aid of enzyme-labeled, fluorescencelabeled, chemiluminescence-labeled, biotin-labeled or radiolabeled antibodies against the epitope-bound antibodies.

16. An analytical method for the detection and/or for the determination of HCMV, which comprises employing as specific step a hybridization reaction which uses at least one nucleic acid probe which in its specific part is complementary to at least one of the DNA sequences as claimed in claim 8 but not to the complete XhoII-PstI fragment contained in the vector pXP1.

17. A process for producing an immunological assay kit for the detection and/or determination of HCMV antibodies, wherein the assay kit contains one or more supports on which one or more epitopes of the peptides or peptide mixtures as claimed in at least one of claims 1 to 7 are immobilized.

18. The process as claimed in claim 17, wherein the support is selected from the group comprising polystyrene, polyvinyl chloride, polyamide and other synthetic polymers, natural polymers such as cellulose, and derivatized natural polymers such as cellulose acetate and nitrocellulose, and glass, especially as glass fibers.

19. The process as claimed in claim 18, wherein the support is polystyrene.

20. The process as claimed in at least one of claims 17-19, wherein the simultaneous detection and/or the simultaneous determination is carried out with the aid of enzyme-labeled, fluorescence-labeled, chemiluminescence-labeled, biotin-labeled or radiolabeled antibodies against the epitope-bound antibodies.

21. The process as claimed in claim 20, wherein the enzymes alkaline phosphatase and/or horseradish peroxidase are used as enzyme-labeled antibodies.

22. An immunochemical combination method for the detection and/or for the determination of a plurality of different antibody specificities each against different pathogens, wherein one or more peptides as claimed in at least one of claims 1 to 7 and one or more immunoreactive peptides from pathogens other than HCMV are used.

23. The immunochemical combination method as claimed in claim 22, wherein peptides from HSV 1/2, EBV, VZV, HHV6, HAV, HBV, HCV and/or HIV 1/2 or immunoreactive parts thereof are used as immunoreactive peptides from other pathogens.

24. The immunochemical combination method as claimed in claim 22 or 23, which method is carried out differentially.

25. A process for producing a combination assay kit for the detection and/or for the determination of a plurality of different antibody specificities each against different pathogens, wherein one or more peptides as claimed in at least one of claims 1 to 7 and one or more immunoreactive peptides from pathogens other than HCMV are immobilized on one or more supports.

26. A process for preparing a vaccine, wherein at least one peptide or a mixture of peptides prepared as claimed in at least one of claims 1-7 are used.

27. The use of peptides or mixtures of peptides as claimed in at least one of claims 1-7 for producing and obtaining antibodies by immunizing mammals, especially rabbits.

28. The use of the antibodies raised by the method as claimed in claim 27 in a diagnostic method in vitro.

29. The use as claimed in claim 28, wherein the diagnostic method is a heterogeneous immunoassay.

## Revendications

1. Peptides réagissant spécifiquement avec des anticorps dirigés contre HCMV (cytomégalovirus humain), **caractérisés en ce qu'**ils contiennent au moins une des séquences d'aminoacides suivantes ASₑ-gly ala gly ala ala ile leu-BSₘ     (peptide 1) CS_n-arg ala trp ala leu-DS_o (peptide 2) et/ou ES_p-ala ser arg asp ala ala-FS_q (peptide 3) AS, BS, CS, DS, ES et FS représentant, indépendamment les uns des autres, un aminoacide quelconque et
e représentant, indépendamment les uns des autres, des nombres entiers allant de 0 à 22,
m représentant, indépendamment les uns des autres, des nombres entiers allant de 0 à 25,
n et o représentant, indépendamment les uns des autres, des nombres entiers allant de 0 à 18, et
p et q représentant, indépendamment les uns des autres, des nombres entiers allant de 0 à 11,
étant entendu qu'ils ne sont pas identiques aux peptides n° 53 et 56 selon Novak et coll. (1991), *J. Gen. Virol.* **72**, p. 1409-1413.

2. Peptides selon la revendication 1, **caractérisés en ce qu'**ils contiennent au moins une des séquences d'aminoacides suivantes, à savoir
en ce qui concerne le peptide 1 : 
1.1
ala tyr lys phe glu gln pro thr leu thr phe gly ala gly val asn val pro ala
gly ala gly ala ala ile leu
1.2
phe glu gln pro thr leu thr phe gly ala gly val asn val pro ala gly ala gly
ala ala ile leu thr pro thr pro val
1.3
phe glu gln pro thr leu thr phe gly ala gly val asn val pro ala gly ala gly
ala ala ile leu thr pro thr pro val asn pro ser thr ala
 
1.4
phe glu gln pro thr thr leu thr phe gly ala gly val asn val pro ala gly ala
gly ala ala ile leu thr pro thr pro val asn pro ser thr ala pro ala pro ala
1.5
pro thr leu thr phe gly ala gly val asn val pro ala gly ala gly ala ala ile
leu thr pro thr pro val asn pro ser thr ala pro ala pro ala
1.6
pro thr leu thr phe gly ala gly val asn val pro ala gly ala gly ala ala ile
leu thr pro thr pro val asn pro ser thr ala pro ala pro ala pro NH2
1.7
val asn val pro ala gly ala gly ala ala ile leu thr pro thr pro val asn pro
1.8
pro ala gly ala gly ala ala ile leu thr pro thr pro val asn pro ser thr ala
pro ala
1.9
val asn val pro ala gly ala gly ala ala ile leu thr pro thr pro val asn pro
ser thr ala pro ala pro ala pro thr pro thr phe
1.10
pro ala gly ala gly ala ala ile leu thr pro thr üpro val asn pro ser thr ala
pro ala pro ala pro thr pro thr phe ala gly thr
1.11
gly ala gly val asn val pro ala gly ala gly ala ala ile leu thr pro thr pro val
asn pro ser thr ala pro ala pro ala pro NH2
1.12
gly ala gly ala ala ile leu thr pro thr pro val asn pro ser thr ala pro ala
pro ala pro thr pro thr phe ala gly thr gln thr pro
1.13
ala tyr lys phe glu gln pro thr leu thr phe gly ala gly val asn val pro ala
gly ala gly ala ala ile leu thr pro thr pro val asn pro ser thr ala pro ala
pro ala pro
1.14
ala tyr lys phe glu gln pro thr leu thr phe gly ala gly val asn val pro ala
gly ala gly ala ala ile leu thr pro thr pro val asn pro ser thr ala pro ala
pro ala pro thr pro thr phe ala gly thr gln thr pro
1.15
ala tyr lys phe glu gln pro thr leu thr phe gly ala gly val asn val pro ala
gly ala gly ala ala ile leu thr pro thr pro val asn pro ser thr ala pro ala
pro ala pro NH2
 
1.16
gly ala gly val asn val pro ala gly ala gly ala ala ile leu thr pro thr pro val
asn pro ser thr ala pro ala pro ala
 en ce qui concerne le peptide 2 : 
2.1
asp met asn pro ala asn trp pro arg glu arg ala trp ala leu lys asn pro
his leu ala tyr asn pro phe
2.2
asp met asn pro ala asn trp pro arg glu arg ala trp ala leu lys asn pro
his leu
2.3
pro ala asn trp pro arg glu arg ala trp ala leu lys asn pro his leu
2.4
pro ala asn trp pro arg glu arg ala trp ala leu lys asn pro his leu ala tyr
asn
2.5
trp pro arg glu arg ala trp ala leu lys asn pro his leu ala tyr asn pro phe
arg
2.6
glu arg ala trp ala leu lys asn pro his leu ala tyr asn pro phe arg met pro
thr thr
2.7
asp met asn pro ala asn trp pro arg glu arg ala trp ala leu
2.8
ala asn trp pro arg glu arg ala trp ala leu lys asn
pro his leu ala
2.9
asp met asn pro ala asn trp pro arg glu arg ala trp ala leu lys asn pro
his leu ala tyr asn pro phe arg met pro thr thr ser thr ala
2.10
arg ala trp ala leu lys asn pro his leu ala tyr asn pro phe
 en ce qui concerne le peptide 3 :

3.1
pro arg ala ala val thr gln thr ala ser arg asp ala ala
3.2
ala ser arg asp ala ala asp glu val trp ala leu arg asp gln thr ala

3. Peptide selon la revendication 1 ou 2, **caractérisé en ce qu'**il est modifié de la façon suivante :
a) attachement à l'extrémité amino-terminale ou carboxy-terminale de 1 à 40, de préférence, 1 à 20 ou, en particulier, 1 à 10 aminoacides, la cystéine de préférence, pour le raccordement de peptides entre eux ou à un support ; ou
b) remplacement d'un aminoacide par un autre aminoacide choisi dans les groupes suivants : Gly, Ala ; Val, Ile, Leu ; Asp, Glu ; Asn, Gln ; Ser, Thr ; Lys, Arg ; Phe, Tyr ; Ala, Ser ; Ala, Thr ; Ala, Val ; Ala, Pro ; Ala, Glu ; Leu, Gln ; Gly, Phe ; Ile, Ser et Ile, Met ; ou
c) addition d'une séquence hydrophobe, comprenant 2 à 20 aminoacides hydrophobes, par exemple, Phe-Ala-Phe-Ala-Phe, pour l'amélioration des propriétés d'adsorption du peptide sur un support.

4. Peptides selon au moins l'une des revendications 1 à 3, **caractérisés en ce qu'**ils sont fixés sur un support.

5. Mélanges de peptides, contenant les peptides selon au moins l'une des revendications 1 à 4.

6. Mélanges de peptides selon la revendication 5, **caractérisés en ce que** les peptides sont raccordés les uns aux autres directement ou par l'intermédiaire d'un espaceur.

7. Peptides ou mélanges de peptides selon au moins l'une des revendications 1 à 6, **caractérisés en ce qu'**ils sont synthétisés par une méthode de la chimie des peptides.

8. Fragments d'ADN qui codent pour au moins l'un des peptides selon l'une quelconque des revendications 1 à 3.

9. Procédé immunochimique pour la détection et/ou la détermination d'anticorps de HCMV à l'aide de peptides selon au moins l'une des revendications 1 à 7.

10. Procédé selon la revendication 9, **caractérisé en ce que** les peptides sont raccordés les uns aux autres directement ou par l'intermédiaire d'un espaceur.

11. Procédé selon la revendication 10, **caractérisé en ce que** l'espaceur est l'albumine de sérum humain et/ou la polylysine.

12. Procédé selon au moins l'une des revendications 9 à 11, **caractérisé en ce que** les peptides sont fixés sur un support.

13. Procédé selon la revendication 12, **caractérisé en ce que** le support est choisi dans l'ensemble constitué par le polystyrène, le poly(chlorure de vinyle), le polyamide et d'autres polymères synthétiques, des polymères naturels tels que la cellulose ainsi que des polymères naturels modifiés, tels que l'acétate de cellulose et la nitrocellulose, et également le verre, en particulier sous forme de fibres de verre.

14. Procédé selon la revendication 13, **caractérisé en ce que** le support est le polystyrène.

15. Procédé selon au moins l'une des revendications 9 à 14, **caractérisé en ce que** la détection et/ou la détermination de l'anticorps lié à un épitope s'effectue à l'aide d'anticorps dirigés contre les anticorps liés à des épitopes, marqués par une enzyme, marqués par fluorescence, marqués par chimiluminescence, marqués à la biotine ou radiomarqués.

16. Procédé analytique pour la détection et/ou la détermination de HCMV, **caractérisé en ce qu'**on utilise comme étape spécifique une réaction d'hybridation, dans laquelle on utilise au moins une sonde d'acide nucléique qui, dans sa partie spécifique, est complémentaire d'au moins l'une des séquences d'ADN selon la revendication 8, mais non du fragment *Xho*II-*Pst*I complet contenu dans le vecteur pXP1.

17. Nécessaire d'essai immunologique pour la détection et/ou la détermination d'anticorps contre HCMV, dans lequel le nécessaire d'essai contient un ou plusieurs supports sur le(s)quel(s) sont fixés un ou plusieurs épitopes des peptides ou mélanges de peptides selon au moins l'une des revendications 1 à 7.

18. Nécessaire d'essai selon la revendication 17, **caractérisé en ce qu** le support est choisi dans l'ensemble constitué par le polystyrène, le poly(chlorure de vinyle), le polyamide et d'autres polymères synthétiques, des polymères naturels tels que la cellulose ainsi que des polymères naturels dérivés, tels que l'acétate de cellulose et la nitrocellulose, et également le verre, en particulier sous forme de fibres de verre.

19. Nécessaire d'essai selon la revendication 18, **caractérisé en ce que** le support est le polystyrène.

20. Nécessaire d'essai selon au moins l'une des revendications 17 à 19, **caractérisé en ce que** la détection simultanée et/ou la détermination simultanée s'effectue(nt) à l'aide d'anticorps dirigés contre les anticorps liés à des épitopes, marqués par une enzyme, marqués par fluorescence, marqués par chimiluminescence, marqués à la biotine ou radiomarqués.

21. Nécessaire d'essai selon la revendication 20, **caractérisé en ce que** comme enzyme utilisée pour marquer les anticorps, on utilise les enzymes phosphatase alcaline et/ou peroxydase de raifort.

22. Procédé immunochimique combiné pour la détection et/ou la détermination de plusieurs spécificités d'anticorps différentes contre, respectivement, différents agents pathogènes, dans lequel on utilise un ou plusieurs peptides selon au moins l'une des revendications 1 à 7, et un ou plusieurs peptides immunoréactifs d'autres agents pathogènes que le HCMV.

23. Procédé immunochimique combiné selon la revendication 22, **caractérisé en ce que** comme peptides immunoréactifs d'autres agents pathogènes, on utilise des peptides de HSV 1/2, EBV, VZV, HHV6, HAV, HBV, HCV et/ou HIV 1/2 ou des parties immunoréactives de ceux-ci.

24. Procédé immunochimique combiné selon la revendication 22 ou 23, **caractérisé en ce que** le procédé est effectué de manière à différencier.

25. Nécessaire d'essai combiné pour la détection et/ou la détermination de plusieurs spécificités d'anticorps différentes contre, respectivement, des agents pathogènes différents, dans lequel un ou plusieurs peptides selon au moins l'une des revendications 1 à 7 et un ou plusieurs peptides immunoréactifs d'autres agents pathogènes que le HCMV sont fixés sur un ou plusieurs supports.

26. Vaccin, contenant au moins un peptide ou un mélange de peptides selon au moins l'une des revendications 1 à 7.

27. Utilisation de peptides ou de mélanges de peptides selon au moins l'une des revendications 1 à 7, pour la production et l'obtention d'anticorps par immunisation de mammifères, en particulier du lapin.

28. Utilisation des anticorps produits par le procédé selon la revendication 27, dans un procédé de diagnostic in vitro.

29. Utilisation selon la revendication 28, **caractérisé en ce que** le procédé de diagnostic est un essai immunologique hétérogène.

## Revendications (Revendications pour l'(les) Etat(s) contractant(s) suivant(s): ES)

1. Procédé pour la production, par génie génétique ou par une méthode de la chimie des peptide, de peptides réagissant spécifiquement avec des anticorps dirigés contre HCMV (cytomégalovirus humain), **caractérisé en ce qu'**ils contiennent au moins une des séquences d'aminoacides suivantes ASₑ-gly ala gly ala ala ile leu-BSₘ (peptide 1) CSₙ-arg ala trp ala leu-DSₒ (peptide 2) et/ou ES_p-ala ser arg asp ala ala-FS_q (peptide 3) AS, BS, CS, DS, ES et FS représentant, indépendamment les uns des autres, un aminoacide quelconque et
e représentant, indépendamment les uns des autres, des nombres entiers allant de 0 à 22,
m représentant, indépendamment les uns des autres, des nombres entiers allant de 0 à 25,
n et o représentant, indépendamment les uns des autres, des nombres entiers allant de 0 à 18, et
p et q représentant, indépendamment les uns des autres, des nombres entiers allant de 0 à 11,
étant entendu qu'ils ne sont pas identiques aux peptides n° 53 et 56 selon Novak et coll. (1991), *J. Gen. Virol*. ***72**,* p. 1409-1413.

2. Procédé selon la revendication 1, **caractérisé en ce que** les peptides contiennent au moins une des séquences d'aminoacides suivantes, à savoir
en ce qui concerne le peptide 1 : 
1.1
ala tyr lys phe glu gln pro thr leu thr phe gly ala gly val asn val pro ala
gly ala gly ala ala ile leu
1.2
phe glu gln pro thr leu thr phe gly ala gly val asn val pro ala gly ala gly
ala ala ile leu thr pro thr pro val
1.3
phe glu gln pro thr leu thr phe gly ala gly val asn val pro ala gly ala gly
ala ala ile leu-thr pro thr pro val asn pro ser thr ala [?]
 
1.4
phe glu gln pro thr thr leu thr phe gly ala gly val asn val pro ala gly ala
gly ala ala ile leu thr pro thr pro val asn pro ser thr ala pro ala pro ala
1.5
pro thr leu thr phe gly ala gly val asn val pro ala gly ala gly ala ala ile
leu thr pro thr pro val asn pro ser thr ala pro ala pro ala
1.6
pro thr leu thr phe gly ala gly val asn val pro ala gly ala gly ala ala ile
leu thr pro thr pro val asn pro ser thr ala pro ala pro ala pro NH2
1.7
val asn val pro ala gly ala gly ala ala ile leu thr pro thr pro val asn pro
1.8
pro ala gly ala gly ala ala ile leu thr pro thr pro val asn pro ser thr ala
pro ala
1.9
val asn val pro ala gly ala gly ala ala ile leu thr pro thr pro val asn pro
ser thr ala pro ala pro ala pro thr pro thr phe
1.10
pro ala gly ala gly ala ala ile leu thr pro thr pro val asn pro ser thr ala
pro ala pro ala pro thr pro thr phe ala gly thr
1.11
gly ala gly val asn val pro ala gly ala gly ala ala ile leu thr pro thr pro val
asn pro ser thr ala pro ala pro ala pro NH2
1.12
gly ala gly ala ala ile leu thr pro thr pro val asn pro ser thr ala pro ala
pro ala pro thr pro thr phe ala gly thr gln thr pro
1.13
ala tyr lys phe glu gln pro thr leu thr phe gly ala gly val asn val pro ala
gly ala gly ala ala ile leu thr pro thr pro val asn pro ser thr ala pro ala
pro ala pro
1.14
ala tyr lys phe glu gln pro thr leu thr phe gly ala gly val asn val pro ala
gly ala gly ala ala ile leu thr pro thr pro val asn pro ser thr ala pro ala
pro ala pro thr pro thr phe ala gly thr gln thr pro
1.15
ala tyr lys phe glu gln pro thr leu thr phe gly ala gly val asn val pro ala
gly ala gly ala ala ile leu thr pro thr pro val asn pro ser thr ala pro ala
pro ala pro NH2
 
1.16
gly ala gly val asn val pro ala gly ala gly ala ala ile leu thr pro thr pro val
asn pro ser thr ala pro ala pro ala
 en ce qui concerne le peptide 2 : 
2.1
asp met asn pro ala asn trp pro arg glu arg ala trp ala leu lys asn pro
his leu ala tyr asn pro phe
2.2
asp met asn pro ala asn trp pro arg glu arg ala trp ala leu lys asn pro
his leu
2.3
pro ala asn trp pro arg glu arg ala trp ala leu lys asn pro his leu
2.4
pro ala asn trp pro arg glu arg ala trp ala leu lys asn pro his leu ala tyr
asn
2.5
trp pro arg glu arg ala trp ala leu lys asn pro his leu ala tyr asn pro phe
arg
2.6
glu arg ala trp ala leu lys asn pro his leu ala tyr asn pro phe arg met pro
thr thr
2.7
asp met asn pro ala asn trp pro arg glu arg ala trp ala leu
2.8
ala asn trp pro arg glu arg ala trp ala leu lys asn
pro his leu ala
2.9
asp met asn pro ala asn trp pro arg glu arg ala trp ala leu lys asn pro
his leu ala tyr asn pro phe arg met pro thr thr ser thr ala
2.10
arg ala trp ala leu lys asn pro his leu ala tyr asn pro phe
 en ce qui concerne le peptide 3 :

3.1
pro arg ala ala val thr gln thr ala ser arg asp ala ala
3.2
ala ser arg asp ala ala asp glu val trp ala leu arg asp gln thr ala

3. Procédé selon la revendication 1 ou 2, **caractérisé en ce que** le peptide est modifié de la façon suivante :
a) attachement à l'extrémité amino-terminale ou carboxy-terminale de 1 à 40, de préférence, 1 à 20 ou, en particulier, 1 à 10 aminoacides, la cystéine de préférence, pour le raccordement de peptides entre eux ou à un support ; ou
b) remplacement d'un aminoacide par un autre aminoacide choisi dans les groupes suivants : Gly, Ala ; Val, Ile, Leu ; Asp, Glu ; Asn, Gln ; Ser, Thr ; Lys, Arg ; Phe, Tyr ; Ala, Ser ; Ala, Thr ; Ala, Val ; Ala, Pro ; Ala, Glu ; Leu, Gln ; Gly, Phe ; Ile, Ser et Ile, Met ; ou
c) addition d'une séquence hydrophobe, comprenant 2 à 20 aminoacides hydrophobes, par exemple, Phe-Ala-Phe-Ala-Phe, pour l'amélioration des propriétés d'adsorption du peptide sur un support.

4. Procédé selon au moins l'une des revendications 1 à 3, **caractérisé en ce que** les peptides sont fixés sur un support.

5. Procédé pour la préparation de mélanges de peptides, **caractérisé en ce qu'**on utilise des peptides préparés par un procédé selon au moins l'une des revendications 1 à 4.

6. Procédé selon la revendication 5, **caractérisé en ce que** les peptides sont raccordés les uns aux autres directement ou par l'intermédiaire d'un espaceur.

7. Procédé selon au moins l'une des revendications 1 à 6, **caractérisé en ce que** les peptides sont synthétisés par une méthode de la chimie des peptides.

8. Procédé pour la préparation de fragments d'ADN qui codent pour au moins l'un des peptides selon l'une quelconque des revendications 1 à 3, **caractérisé en ce que** les fragments d'ADN sont synthétisés par génie génétique ou par une méthode de la chimie des peptides.

9. Procédé immunochimique pour la détection et/ou la détermination d'anticorps de HCMV à l'aide de peptides selon au moins l'une des revendications 1 à 7.

10. Procédé selon la revendication 9, **caractérisé en ce que** les peptides sont raccordés les uns aux autres directement ou par l'intermédiaire d'un espaceur.

11. Procédé selon la revendication 10, **caractérisé en ce que** l'espaceur est l'albumine de sérum humain et/ou la polylysine.

12. Procédé selon au moins l'une des revendications 9 à 11, **caractérisé en ce que** les peptides sont fixés sur un support.

13. Procédé selon la revendication 12, **caractérisé en ce que** le support est choisi dans l'ensemble constitué par le polystyrène, le poly(chlorure de vinyle), le polyamide et d'autres polymères synthétiques, des polymères naturels tels que la cellulose ainsi que des polymères naturels modifiés, tels que l'acétate de cellulose et la nitrocellulose, et également le verre, en particulier sous forme de fibres de verre.

14. Procédé selon la revendication 13, **caractérisé en ce que** le support est le polystyrène.

15. Procédé selon au moins l'une des revendications 9 à 14, **caractérisé en ce que** la détection et/ou la détermination de l'anticorps lié à un épitope s'effectue à l'aide d'anticorps dirigés contre les anticorps liés à des épitopes, marqués par une enzyme, marqués par fluorescence, marqués par chimiluminescence, marqués à la biotine ou radiomarqués.

16. Procédé analytique pour la détection et/ou la détermination de HCMV, **caractérisé en ce qu'**on utilise comme étape spécifique une réaction d'hybridation, dans laquelle on utilise au moins une sonde d'acide nucléique qui, dans sa partie spécifique, est complémentaire d'au moins l'une des séquences d'ADN selon la revendication 8, mais non du fragment *Xho*II-*Pst*I complet contenu dans le vecteur pXP1.

17. Procédé pour la préparation d'un nécessaire d'essai immunologique pour la détection et/ou la détermination d'anticorps contre HCMV, dans lequel le nécessaire d'essai contient un ou plusieurs supports sur le(s)quel(s) sont fixés un ou plusieurs épitopes des peptides ou mélanges de peptides selon au moins l'une des revendications 1 à 7.

18. Procédé selon la revendication 17, **caractérisé en ce qu** le support est choisi dans l'ensemble. constitué par le polystyrène, le poly(chlorure de vinyle), le polyamide et d'autres polymères synthétiques, des polymères naturels tels que la cellulose ainsi que des polymères naturels dérivés, tels que l'acétate de cellulose et la nitrocellulose, et également le verre, en particulier sous forme de fibres de verre.

19. Procédé selon la revendication 18, **caractérisé en ce que** le support est le polystyrène.

20. Procédé selon au moins l'une des revendications 17 à 19, **caractérisé en ce que** la détection simultanée et/ou la détermination simultanée s'effectue(nt) à l'aide d'anticorps dirigés contre les anticorps liés à des épitopes, marqués par une enzyme, marqués par fluorescence, marqués par chimiluminescence, marqués à la biotine ou radiomarqués.

21. Procédé selon la revendication 20, **caractérisé en ce que** comme enzyme utilisée pour marquer les anticorps, on utilise les enzymes phosphatase alcaline et/ou peroxydase de raifort.

22. Procédé immunochimique combiné pour la détection et/ou la détermination de plusieurs spécificités d'anticorps différentes contre, respectivement, différents agents pathogènes, dans lequel on utilise un ou plusieurs peptides selon au moins l'une des revendications 1 à 7, et un ou plusieurs peptides immunoréactifs d'autres agents pathogènes que le HCMV.

23. Procédé immunochimique combiné selon la revendication 22, **caractérisé en ce que** comme peptides immunoréactifs d'autres agents pathogènes, on utilise des peptides de HSV 1/2, EBV, VZV, HHV6, HAV, HBV, HCV et/ou HIV 1/2 ou des parties immunoréactives de ceux-ci.

24. Procédé immunochimique combiné selon la revendication 22 ou 23, **caractérisé en ce que** le procédé est effectué de manière à différencier.

25. Procédé pour la préparation d'un nécessaire d'essai combiné pour la détection et/ou la détermination de plusieurs spécificités d'anticorps différentes contre, respectivement, des agents pathogènes différents, dans lequel un ou plusieurs peptides selon au moins l'une des revendications 1 à 7 et un ou plusieurs peptides immunoréactifs d'autres agents pathogènes que le HCMV sont fixés sur un ou plusieurs supports.

26. Procédé pour la préparation d'un vaccin, **caractérisé en ce qu'**on utilise au moins un peptide ou un mélange de peptides préparé selon au moins l'une des revendications 1 à 7.

27. Utilisation de peptides ou de mélanges de peptides selon au moins l'une des revendications 1 à 7, pour la production et l'obtention d'anticorps par immunisation de mammifères, en particulier du lapin.

28. Utilisation des anticorps produits par le procédé selon la revendication 27, dans un procédé de diagnostic in vitro.

29. Utilisation selon la revendication 28, **caractérisé en ce que** le procédé de diagnostic est un essai immunologique hétérogène.
